# EUROPEAN PATENT APPLICATION

(11) **EP 2 045 329 A1**
(43) Date of publication of application: **08.04.2009**
(21) Application number: 07790827.5
(22) Date of filing: 17.07.2007
(51) Int. Cl.: C12N 15/31, A61K 39/00, A61K 39/09, A61K 39/104, A61P 31/04, C07K 14/21, C12N 1/15, C12N 1/19, C12N 1/21, C12N 5/10, C12P 21/02

(54) **FLAGELLIN MUTANT VACCINE**

(30) Priority: 21.07.2006 JP 2006199361; 29.01.2007 JP 2007017446
(71) Applicant: Yokohama City University, Yokohama-shi Kanagawa 236-0027 (JP)
(72) Inventor: TAKESHITA, Fumihiko, Yokohama-shi Kanagawa 236-0004 (JP); OKUDA, Kenji, Yokohama-shi Kanagawa 236-0004 (JP); SAHA, Sukumar, Yokohama-shi Kanagawa 236-0004 (JP)
(74) Representative: Woods, Geoffrey Corlett
(86) International application number: PCT/JP2007/064065
(87) International publication number: WO 2008/010478

(57) **Abstract**

The present invention provides a vaccine which effectively induces protective immune response particularly against flagellated pathogens (such as *Pseudomonas aeruginasa).*

A flagellin mutant into which a mutation has been introduced at least at one site in the Toll-like receptor 5 activation domain of a corresponding wild-type flagellin to thereby attenuate the ability to activate Toll-like receptor 5. A vaccine targeting the flagellin mutant.

SEQUENCE LISTING

<110> Yokohama City University

<120> Vaccines targeting mutant flagellins

<130> FP-084PCT

<150> JP P2006-199361
<151> 2006-07-21

<150> JP P2007-017446
<151> 2007-01-29

<160> 6

<170> PatentIn version 3.1

<210> 1
<211> 1467
<212> DNA
<213> Pseudomonas aeruginosa

<400> 1
atggccctta cagtcaacac gaacattgct tccctgaaca ctcagcgcaa cctgaatgct 60

tcttccaacg acctcaacac ctcgttggag cgtctgacca ccggctaccg catcaacagt 120

gccaaggacg atgctgccgg cctgcagatc tccaaccgcc tgtccaacca gatcagcggt 180

ctgaacgttg ccacccgcaa cgccaacgac ggcatctccc tggcgcagac cgctgaaggt 240

gccctgcagc agtccaccaa tatcctgcag cgtatccgcg acctggccct gcaatccgcc 300

aacggctcca acagcgacgc cgaccgtgcc gccctgcaga aagaagtcgc tgcgcaacag 360

gccgaactga cccgtatctc cgataccacc accttcggtg gccgcaagct gctcgacggc 420

tccttcggca ccaccagctt ccaggtcggt tccaacgcct acgagaccat tgacatcagc 480

ctgcagaatg cctctgccag cgccatcggt tcttaccagg tcggcagcaa cggcgcgggt 540

accgtcgcca gcgtagcggg caccgcgacc gcttcgggca tcgcctcggg caccgtcaac 600

ctggtcggtg gcggtcaggt gaagaacatc gccatcgccg ccggcgatag cgccaaggcc 660

atcgccgaga agatggacgg tgcgatcccg aacctgtcgg ctcgtgcccg taccgtgttc 720

accgctgatg tcagcggcgt gaccggtggt tcgctgaact tcgacgtaac cgttggcagc 780

aacaccgtga gcctggcagg cgtgacctcc actcaagatc tggccgacca actgaactcc 840

aactcgtcga agctgggcat cactgccagc atcaacgaca agggtgtact gaccatcacc 900

tccgctaccg gcgagaacgt caagttcggt gcgcagaccg gtaccgctac tgccggtcag 960

gtcgcagtga aggtccaggg ttccgacggc aagttcgaag cggccgccaa gaacgtggta 1020

gctgccggta ctgccgctac caccaccatc gtgaccggct acgtgcaact gaactcgccg 1080

accgcctact cggtcagcgg taccggcacc caggcttcgc aggtcttcgc caacgccagc 1140

gccgcgcaga agagcagcgt tgccagcgtc gacatctcca ctgccgacgg cgcccagaac 1200

gccatcgcgg tagtcgataa cgccctggct gcgatcgacg cccagcgtgc tgacctcggt 1260

gctgttcaga accgcttcaa gaacactatc gacaacctga ccaacatctc ggaaaacgct 1320

accaacgctc gtagccgcat caaggacacc gacttcgctg ccgaaaccgc ggcgctgtcg 1380

aagaaccagg tgctgcaaca ggccggtacc gcgatcctgg cccaggccaa ccagctgccg 1440

caggcggtcc tgagcctgct gcgctaa 1467

<210> 2
<211> 487
<212> PRT
<213> Pseudomonas aeruginosa

<400> 2

Ala Leu Thr Val Asn Thr Asn Ile Ala Ser Leu Asn Thr Gln Arg Asn
1               5                   10                  15

Leu Asn Ala Ser Ser Asn Asp Leu Asn Thr Ser Leu Glu Arg Leu Thr
            20                  25                  30

Thr Gly Tyr Arg Ile Asn Ser Ala Lys Asp Asp Ala Ala Gly Leu Gln
        35                  40                  45

Ile Ser Asn Arg Leu Ser Asn Gln Ile Ser Gly Leu Asn Val Ala Thr
    50                  55                  60

Arg Asn Ala Asn Asp Gly Ile Ser Leu Ala Gln Thr Ala Glu Gly Ala
65                  70                  75                  80

Leu Gln Gln Ser Thr Asn Ile Leu Gln Arg Ile Arg Asp Leu Ala Leu
                85                  90                  95

Gln Ser Ala Asn Gly Ser Asn Ser Asp Ala Asp Arg Ala Ala Leu Gln
            100                 105                 110

Lys Glu Val Ala Ala Gln Gln Ala Glu Leu Thr Arg Ile Ser Asp Thr
        115                 120                 125

Thr Thr Phe Gly Gly Arg Lys Leu Leu Asp Gly Ser Phe Gly Thr Thr
    130                 135                 140

Ser Phe Gln Val Gly Ser Asn Ala Tyr Glu Thr Ile Asp Ile Ser Leu
145                 150                 155                 160

Gln Asn Ala Ser Ala Ser Ala Ile Gly Ser Tyr Gln Val Gly Ser Asn
                165                 170                 175

Gly Ala Gly Thr Val Ala Ser Val Ala Gly Thr Ala Thr Ala Ser Gly
            180                 185                 190

Ile Ala Ser Gly Thr Val Asn Leu Val Gly Gly Gly Gln Val Lys Asn
        195                 200                 205

Ile Ala Ile Ala Ala Gly Asp Ser Ala Lys Ala Ile Ala Glu Lys Met
    210                 215                 220

Asp Gly Ala Ile Pro Asn Leu Ser Ala Arg Ala Arg Thr Val Phe Thr
225                 230                 235                 240

Ala Asp Val Ser Gly Val Thr Gly Gly Ser Leu Asn Phe Asp Val Thr
                245                 250                 255

Val Gly Ser Asn Thr Val Ser Leu Ala Gly Val Thr Ser Thr Gln Asp
            260                 265                 270

Leu Ala Asp Gln Leu Asn Ser Asn Ser Ser Lys Leu Gly Ile Thr Ala
        275                 280                 285

Ser Ile Asn Asp Lys Gly Val Leu Thr Ile Thr Ser Ala Thr Gly Glu
    290                 295                 300

Asn Val Lys Phe Gly Ala Gln Thr Gly Thr Ala Thr Ala Gly Gln Val
305                 310                 315                 320

Ala Val Lys Val Gln Gly Ser Asp Gly Lys Phe Glu Ala Ala Ala Lys
                325                 330                 335

Asn Val Val Ala Ala Gly Thr Ala Ala Thr Thr Thr Ile Val Thr Gly
            340                 345                 350

Tyr Val Gln Leu Asn Ser Pro Thr Ala Tyr Ser Val Ser Gly Thr Gly
        355                 360                 365

Thr Gln Ala Ser Gln Val Phe Ala Asn Ala Ser Ala Ala Gln Lys Ser
    370                 375                 380

Ser Val Ala Ser Val Asp Ile Ser Thr Ala Asp Gly Ala Gln Asn Ala
385                 390                 395                 400

Ile Ala Val Val Asp Asn Ala Leu Ala Ala Ile Asp Ala Gln Arg Ala
                405                 410                 415

Asp Leu Gly Ala Val Gln Asn Arg Phe Lys Asn Thr Ile Asp Asn Leu
            420                 425                 430

Thr Asn Ile Ser Glu Asn Ala Thr Asn Ala Arg Ser Arg Ile Lys Asp
        435                 440                 445

Thr Asp Phe Ala Ala Glu Thr Ala Ala Leu Ser Lys Asn Gln Val Leu
    450                 455                 460

Gln Gln Ala Gly Thr Ala Ile Leu Ala Gln Ala Asn Gln Leu Pro Gln
465                 470                 475                 480

Ala Val Leu Ser Leu Leu Arg
                485

<210>  3
<211>  1467
<212>  DNA
<213>  Artificial

<220>
<223>  DNA encoding mutant flagellin FliC R90A

<400>  3
atggccctta cagtcaacac gaacattgct tccctgaaca ctcagcgcaa cctgaatgct      60

tcttccaacg acctcaacac ctcgttggag cgtctgacca ccggctaccg catcaacagt     120

gccaaggacg atgctgccgg cctgcagatc tccaaccgcc tgtccaacca gatcagcggt     180

ctgaacgttg ccacccgcaa cgccaacgac ggcatctccc tggcgcagac cgctgaaggt     240

gccctgcagc agtccaccaa tatcctgcag gctatccgcg acctggccct gcaatccgcc     300

aacggctcca acagcgacgc cgaccgtgcc gccctgcaga aagaagtcgc tgcgcaacag     360

gccgaactga cccgtatctc cgataccacc accttcggtg gccgcaagct gctcgacggc     420

tccttcggca ccaccagctt ccaggtcggt tccaacgcct acgagaccat tgacatcagc     480

ctgcagaatg cctctgccag cgccatcggt tcttaccagg tcggcagcaa cggcgcgggt     540

accgtcgcca gcgtagcggg caccgcgacc gcttcgggca tcgcctcggg caccgtcaac     600

ctggtcggtg gcggtcaggt gaagaacatc gccatcgccg ccggcgatag cgccaaggcc     660

atcgccgaga agatggacgg tgcgatcccg aacctgtcgg ctcgtgcccg taccgtgttc     720

accgctgatg tcagcggcgt gaccggtggt tcgctgaact tcgacgtaac cgttggcagc     780

aacaccgtga gcctggcagg cgtgacctcc actcaagatc tggccgacca actgaactcc 840

aactcgtcga agctgggcat cactgccagc atcaacgaca agggtgtact gaccatcacc 900

tccgctaccg gcgagaacgt caagttcggt gcgcagaccg gtaccgctac tgccggtcag 960

gtcgcagtga aggtccaggg ttccgacggc aagttcgaag cggccgccaa gaacgtggta 1020

gctgccggta ctgccgctac caccaccatc gtgaccggct acgtgcaact gaactcgccg 1080

accgcctact cggtcagcgg taccggcacc caggcttcgc aggtcttcgc caacgccagc 1140

gccgcgcaga agagcagcgt tgccagcgtc gacatctcca ctgccgacgg cgcccagaac 1200

gccatcgcgg tagtcgataa cgccctggct gcgatcgacg cccagcgtgc tgacctcggt 1260

gctgttcaga accgcttcaa gaacactatc gacaacctga ccaacatctc ggaaaacgct 1320

accaacgctc gtagccgcat caaggacacc gacttcgctg ccgaaaccgc ggcgctgtcg 1380

aagaaccagg tgctgcaaca ggccggtacc gcgatcctgg cccaggccaa ccagctgccg 1440

caggcggtcc tgagcctgct gcgctaa 1467

<210> 4
<211> 487
<212> PRT
<213> Artificial

<220>
<223> mutant flagellin FliC R90A

<400> 4

Ala Leu Thr Val Asn Thr Asn Ile Ala Ser Leu Asn Thr Gln Arg Asn
1               5                   10                  15

Leu Asn Ala Ser Ser Asn Asp Leu Asn Thr Ser Leu Glu Arg Leu Thr
            20                  25                  30

Thr Gly Tyr Arg Ile Asn Ser Ala Lys Asp Asp Ala Ala Gly Leu Gln
        35                  40                  45

Ile Ser Asn Arg Leu Ser Asn Gln Ile Ser Gly Leu Asn Val Ala Thr
    50                  55                  60

Arg Asn Ala Asn Asp Gly Ile Ser Leu Ala Gln Thr Ala Glu Gly Ala
65                  70                  75                  80

Leu Gln Gln Ser Thr Asn Ile Leu Gln Ala Ile Arg Asp Leu Ala Leu
                85                  90                  95

Gln Ser Ala Asn Gly Ser Asn Ser Asp Ala Asp Arg Ala Ala Leu Gln
            100                 105                 110

Lys Glu Val Ala Ala Gln Gln Ala Glu Leu Thr Arg Ile Ser Asp Thr
        115                 120                 125

Thr Thr Phe Gly Gly Arg Lys Leu Leu Asp Gly Ser Phe Gly Thr Thr
    130                 135                 140

Ser Phe Gln Val Gly Ser Asn Ala Tyr Glu Thr Ile Asp Ile Ser Leu
145                 150                 155                 160

Gln Asn Ala Ser Ala Ser Ala Ile Gly Ser Tyr Gln Val Gly Ser Asn
                165                 170                 175

Gly Ala Gly Thr Val Ala Ser Val Ala Gly Thr Ala Thr Ala Ser Gly
            180                 185                 190

Ile Ala Ser Gly Thr Val Asn Leu Val Gly Gly Gly Gln Val Lys Asn
        195                 200                 205

Ile Ala Ile Ala Ala Gly Asp Ser Ala Lys Ala Ile Ala Glu Lys Met
    210                 215                 220

Asp Gly Ala Ile Pro Asn Leu Ser Ala Arg Ala Arg Thr Val Phe Thr
225                 230                 235                 240

Ala Asp Val Ser Gly Val Thr Gly Gly Ser Leu Asn Phe Asp Val Thr
                245                 250                 255

Val Gly Ser Asn Thr Val Ser Leu Ala Gly Val Thr Ser Thr Gln Asp
            260                 265                 270

Leu Ala Asp Gln Leu Asn Ser Asn Ser Ser Lys Leu Gly Ile Thr Ala
        275                 280                 285

Ser Ile Asn Asp Lys Gly Val Leu Thr Ile Thr Ser Ala Thr Gly Glu
    290                 295                 300

Asn Val Lys Phe Gly Ala Gln Thr Gly Thr Ala Thr Ala Gly Gln Val
305                 310                 315                 320

Ala Val Lys Val Gln Gly Ser Asp Gly Lys Phe Glu Ala Ala Ala Lys
                325                 330                 335

Asn Val Val Ala Ala Gly Thr Ala Ala Thr Thr Thr Ile Val Thr Gly
            340                 345                 350

Tyr Val Gln Leu Asn Ser Pro Thr Ala Tyr Ser Val Ser Gly Thr Gly
        355                 360                 365

Thr Gln Ala Ser Gln Val Phe Ala Asn Ala Ser Ala Ala Gln Lys Ser
    370                 375                 380

Ser Val Ala Ser Val Asp Ile Ser Thr Ala Asp Gly Ala Gln Asn Ala
385                 390                 395                 400

Ile Ala Val Val Asp Asn Ala Leu Ala Ala Ile Asp Ala Gln Arg Ala
                405                 410                 415

Asp Leu Gly Ala Val Gln Asn Arg Phe Lys Asn Thr Ile Asp Asn Leu
            420                 425                 430

Thr Asn Ile Ser Glu Asn Ala Thr Asn Ala Arg Ser Arg Ile Lys Asp
        435                 440                 445

Thr Asp Phe Ala Ala Glu Thr Ala Ala Leu Ser Lys Asn Gln Val Leu
    450                 455                 460

Gln Gln Ala Gly Thr Ala Ile Leu Ala Gln Ala Asn Gln Leu Pro Gln
465                 470                 475                 480

Ala Val Leu Ser Leu Leu Arg
                485

<210>  5
<211>  1185
<212>  DNA
<213>  Pseudomonas aeruginosa

<400>  5
atggccttga ccgtcaacac caacatcgct tcgctgaaca ctcagcggaa cctgaacaac      60

tcttccgcgt cgctgaacac ttcgctgcag cgtctgtcca ccggttcgcg catcaacagc     120

gccaaggacg acgccgccgg cctgcagatc gccaaccgtc tgaccagcca ggtcaacggc     180

ctgaacgtgg ctaccaagaa cgccaacgac ggtatctccc tggcgcagac cgctgaaggc     240

gccctgcagc agtcgaccaa catcctgcag cgtatgcgtg acctgtccct gcagtcggcc     300

aacggctcca acagcgactc cgagcgtacc gctctgaacg gcgaagcgaa gcaactgcag     360

aaagaactgg atcgtatcag caacaccacc accttcggtg gccgcaagct gctcgacggt     420

tccttcggcg tcgccagctt ccaggtgggt tcggccgcca acgaaatcat cagcgtcggc     480

atcgacgaga tgagcgcaga gtcgctgaac ggcacctact tcaaggctga cggcggcggc     540

gcggtcactg ctgcaaccgc ttcgggcacc gtcgacatcg cgatcggcat caccggcggc     600

agcgccgtga acgtcaaggt cgacatgaag ggcaacgaaa ccgccgagca ggcggctgcc     660

aagatcgccg cagcggtcaa cgacgccaac gtcggcatcg gtgccttcag cgacggcgat     720

accatcagct atgtttccaa agctggcaag gatggctccg gtgcgatcac tagcgcggtt     780

tccggcgttg tcatcgctga caccggcagc accggcgtag gcaccgcggc tggcgtagcc 840

ccttccgcta ccgctttcgc caagaccaac gacaccgtcg ccaagatcga catctccacc 900

gcgaaggcgc tcagtcgccg tgctggtgat cgcacgacgg cgatcaagca gatcgacgcc 960

agcgtgccga cctcggtcgc ggtgcagaac cgcttcgaca acaccatcaa caacctgaag 1020

aacatcggtg agaacgtatc ggctgctcgc ggccggatcg aagacaccga cttcgcagcc 1080

gaaaccgcca acctgaccaa gaaccaagtg ctgcaacaag ccggcaccgc gatcctggcc 1140

caggccaacc agctgccgca gtcggttctg agcctgctgc gctaa 1185

<210> 6
<211> 393
<212> PRT
<213> Pseudomonas aeruginosa

<400> 6

Ala Leu Thr Val Asn Thr Asn Ile Ala Ser Leu Asn Thr Gln Arg Asn
1               5                   10                  15

Leu Asn Asn Ser Ser Ala Ser Leu Asn Thr Ser Leu Gln Arg Leu Ser
            20                  25                  30

Thr Gly Ser Arg Ile Asn Ser Ala Lys Asp Asp Ala Ala Gly Leu Gln
        35                  40                  45

Ile Ala Asn Arg Leu Thr Ser Gln Val Asn Gly Leu Asn Val Ala Thr
    50                  55                  60

Lys Asn Ala Asn Asp Gly Ile Ser Leu Ala Gln Thr Ala Glu Gly Ala
65                  70                  75                  80

Leu Gln Gln Ser Thr Asn Ile Leu Gln Arg Met Arg Asp Leu Ser Leu
                85                  90                  95

Gln Ser Ala Asn Gly Ser Asn Ser Asp Ser Glu Arg Thr Ala Leu Asn
            100                 105                 110

Gly Glu Ala Lys Gln Leu Gln Lys Glu Leu Asp Arg Ile Ser Asn Thr
        115                 120                 125

Thr Thr Phe Gly Gly Arg Lys Leu Leu Asp Gly Ser Phe Gly Val Ala
    130                 135                 140

Ser Phe Gln Val Gly Ser Ala Ala Asn Glu Ile Ile Ser Val Gly Ile
145                 150                 155                 160

Asp Glu Met Ser Ala Glu Ser Leu Asn Gly Thr Tyr Phe Lys Ala Asp
                165                 170                 175

Gly Gly Gly Ala Val Thr Ala Ala Thr Ala Ser Gly Thr Val Asp Ile
            180                 185                 190

Ala Ile Gly Ile Thr Gly Gly Ser Ala Val Asn Val Lys Val Asp Met
        195                 200                 205

Lys Gly Asn Glu Thr Ala Glu Gln Ala Ala Ala Lys Ile Ala Ala Ala
    210                 215                 220

Val Asn Asp Ala Asn Val Gly Ile Gly Ala Phe Ser Asp Gly Asp Thr
225                 230                 235                 240

Ile Ser Tyr Val Ser Lys Ala Gly Lys Asp Gly Ser Gly Ala Ile Thr
                245                 250                 255

Ser Ala Val Ser Gly Val Val Ile Ala Asp Thr Gly Ser Thr Gly Val
            260                 265                 270

Gly Thr Ala Ala Gly Val Ala Pro Ser Ala Thr Ala Phe Ala Lys Thr
        275                 280                 285

Asn Asp Thr Val Ala Lys Ile Asp Ile Ser Thr Ala Lys Ala Leu Ser
    290                 295                 300

Arg Arg Ala Gly Asp Arg Thr Thr Ala Ile Lys Gln Ile Asp Ala Ser
305                 310                 315                 320

Val Pro Thr Ser Val Ala Val Gln Asn Arg Phe Asp Asn Thr Ile Asn
                325                 330                 335

Asn Leu Lys Asn Ile Gly Glu Asn Val Ser Ala Ala Arg Gly Arg Ile
            340                 345                 350

Glu Asp Thr Asp Phe Ala Ala Glu Thr Ala Asn Leu Thr Lys Asn Gln
        355                 360                 365

Val Leu Gln Gln Ala Gly Thr Ala Ile Leu Ala Gln Ala Asn Gln Leu
    370                 375                 380

Pro Gln Ser Val Leu Ser Leu Leu Arg
385                 390

## Description

### TECHNICAL FIELD

The present invention relates to a flagellin mutant vaccine.

### BACKGROUND ART

*Pseudomonas aeruginosa,* which is a pathogenic bacterium for opportunistic infections associated with chronic diseases, has become a clinically serious problem because of the occurrence of its drug resistant strains. Although various vaccines have been examined, clinically applicable vaccines have not yet been developed (Non-Patent Document 1).
[Non-Patent Document 1] Holder IA, Pseudomonas immunotherapy: a historical overview, Vaccine 2004; 22(7):831-9

### DISCLOSURE OF THE INVENTION

### PROBLEM FOR SOLUTION BY THE INVENTION

It is an object of the present invention to provide a vaccine, more specifically, a vaccine which effectively induces protective immune response against flagellated pathogens (e.g., *P. aeruginosa).*

### MEANS TO SOLVE THE PROBLEM

Flagellin is a structural component of flagella which are organelles essential for the motility of bacteria. It has been revealed that flagellin activates Toll-like receptor 5 (TLRS)-mediated host's innate immune response. The present inventors have revealed that immunization with a wild-type flagellin induces a neutralizing antibody against this and, as a result, (1) activation of TLR5-mediated host's innate immune response by bacterial flagellin is inhibited and (2) protection against bacteria is remarkably inhibited. Then, the present inventors have created a mutant flagellin FliC R90A with remarkably attenuated TRL5 activation ability, by introducing a site-directed mutation into the TLR5 activation domain of flagellin. A DNA vaccine targeting this mutant flagellin was prepared. Immunization with this vaccine conferred strong protection against homologous flagellin-expressing strains. Besides, immunization with this vaccine also conferred protection against *P. aeruginosa* strains expressing heterologous flagellin. From these results, it has become possible to develop a vaccine capable of conferring strong protection against heterologous flagellin-expressing heterologous *P. aeruginosa* strains, by using FliC R90A as an antigen.

The gist of the present invention is as described below.
(1) A flagellin mutant into which a mutation has been introduced at least at one site in the Toll-like receptor 5 activation domain of a corresponding wild-type flagellin to thereby attenuate the ability to activate Toll-like receptor 5.
(2) The flagellin mutant of (1), wherein the wild-type flagellin is derived from a flagellated pathogen.
(3) The flagellin mutant of (2), wherein the flagellated pathogen is *Pseudomonas aeruginosa.*
(4) The flagellin mutant of any one of (1) to (3), wherein the wild-type flagellin is flagellin FlaA or FliC.
(5) The flagellin mutant of (4), wherein the wild-type flagellin is wild-type flagellin FliC having the amino acid sequence as shown in SEQ ID NO: 2 and the Toll-like receptor 5 activation domain includes a region spanning from position 71 to position 97 of the amino acid sequence as shown in SEQ ID NO: 2.
(6) The flagellin mutant of (5), which has the amino acid sequence as shown SEQ ID NO: 2 wherein arginine at position 90 is substituted with another amino acid.
(7) The flagellin mutant of (6), wherein another amino acid is alanine or glycine.
(8) The flagellin mutant of (1), which is a protein selected from the following (a), (b) and (c):
   (a) a flagellin mutant protein having the amino acid sequence as shown in SEQ ID NO: 2 wherein arginine at position 90 is substituted with alanine;
   (b) a flagellin mutant protein which has the amino acid sequence of the protein of (a) wherein one or more amino acids other than alanine at position 90 are deleted, substituted or added, and whose Toll-like receptor 5 activation ability is attenuated compared to the corresponding ability of wild-type flagellin FliC having the amino acid sequence as shown in SEQ ID NO: 2; and
   (c) a flagellin mutant protein which is encoded by a DNA that hybridizes to a complementary DNA to a DNA encoding the protein of (a) under stringent conditions, and whose Toll-like receptor 5 activation ability is attenuated compared to the corresponding ability of wild-type flagellin FliC having the amino acid sequence as shown in SEQ ID NO: 2.
(9) The flagellin mutant of (8), wherein the DNA encoding the protein of (a) has the polynucleotide sequence as shown in SEQ ID NO: 3.
(10) A DNA encoding the flagellin mutant of any one of (1) to (9).
(11) A vector comprising the DNA of (10).
(12) A transformant comprising the vector of (11).
(13) A method of preparing a flagellin mutant, comprising culturing the transformant of (12).
(14) A vaccine comprising the flagellin mutant of any one of (1) to (9), the DNA of (10) or the vector of (11).
(15) The vaccine of (14), which is against a flagellated pathogen.
(16) The vaccine of (15), wherein the flagellated pathogen is *Pseudomonas aeruginosa.*
(17) The vaccine of any one of (14) to (16), which is used for protection against cystic fibrosis and/or protection of medically compromised patients.
(18) An antibody induced against the flagellin mutant of any one of (1) to (9).
(19) A pharmaceutical composition comprising the antibody of (18).

### EFFECT OF THE INVENTION

The flagellin mutant-targeting vaccine of the present invention is capable of conferring strong protection against flagellated bacteria, in particular *P. aeruginosa.*

Further, an antibody induced against the flagellin mutant of the present invention is capable of effectively eliminating flagellated bacteria, in particular *P. aeruginosa.*

The present specification encompasses the contents disclosed in the specifications and/or the drawings of Japanese Patent Applications No. 2006-199361 and No. 2007-017446 based on which the present patent application claims priority.

### BRIEF DESCRIPTION OF THE DRAWINGS

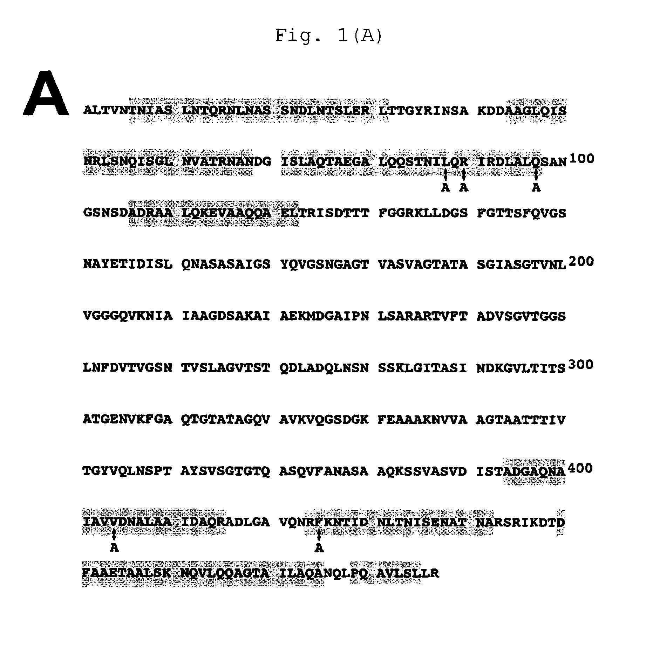

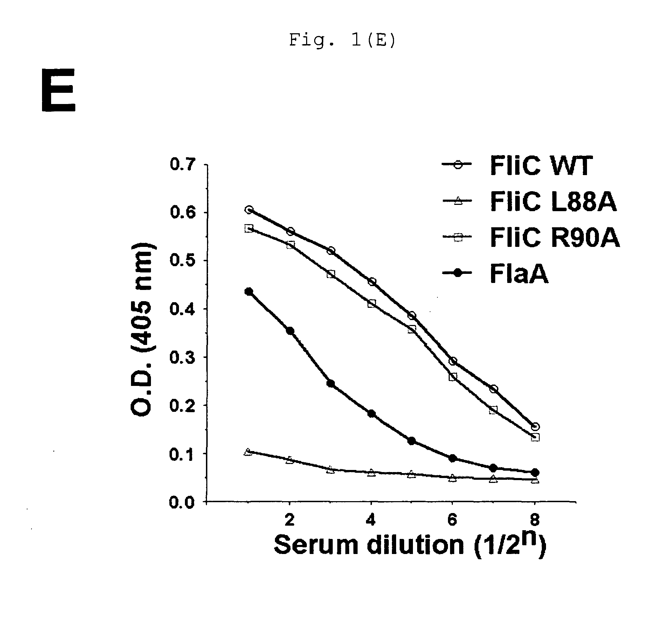

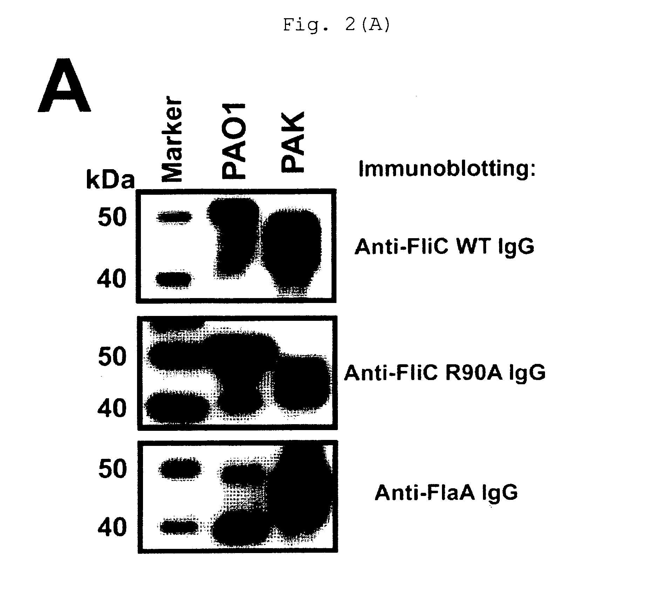

Fig. 1(A) Characterization of flagellin mutants
   Amino acid sequence of *P. aeruginosa* FliC. The domains which form α-helix are shaded with red. It is believed that the conformation formed by these domains constitute the TLR5 activation domain in FliC.

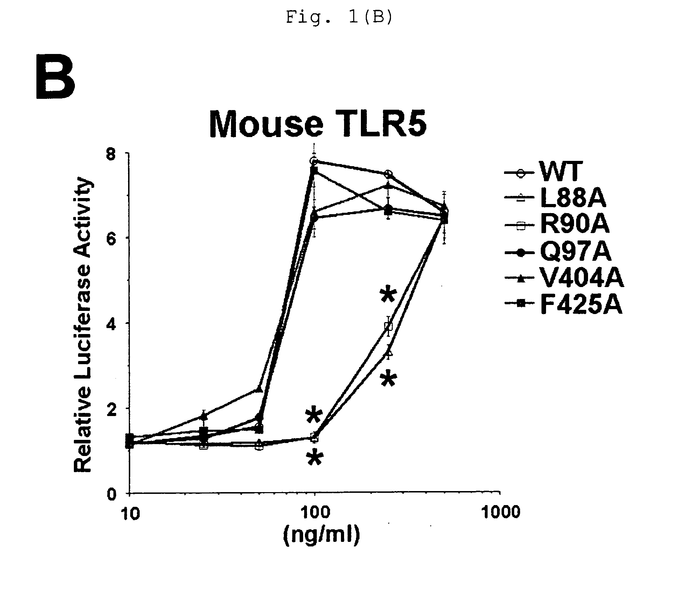

Fig. 1(B) Characterization of flagellin mutants
   HEK293 cells were transiently transfected with mouse TLR5 expression plasmid plus NF-κB-dependent luciferase expression plasmid. Cells were then stimulated with FliC WT or a mutant (L88A, R90A, Q97A, V404A or F425A) at concentrations of 10 to 500 ng/ml and luciferase activity was measured. The graph shows the mean ± SD values. Mark * represents *p*<0.01 by student *t*-test. Two to three independent experiments gave similar results.

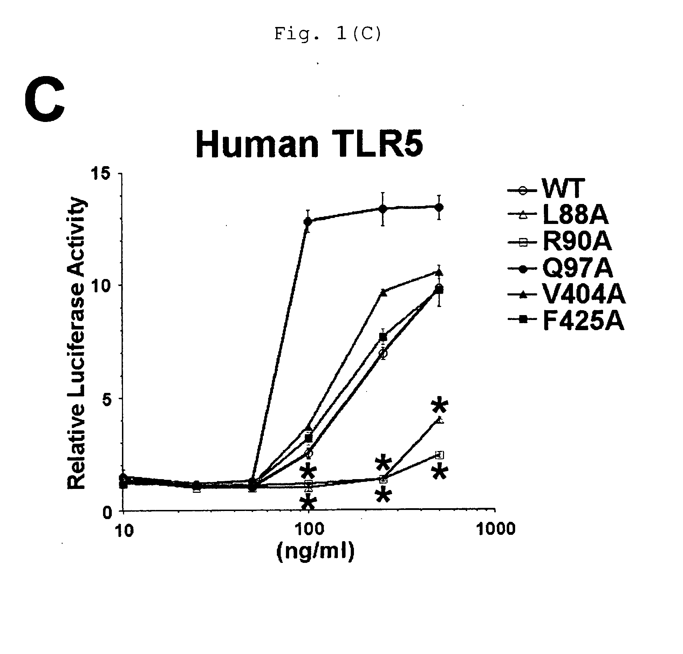

Fig. 1(C) Characterization of flagellin mutants
   HEK293 cells were transiently transfected with human TLR5 expression plasmid plus NF-κB-dependent luciferase expression plasmid. Cells were then stimulated with FliC WT or a mutant (L88A, R90A, Q97A, V404A or F425A) at concentrations of 10 to 500 ng/ml and luciferase activity was measured. The graph shows the mean ± SD values. Mark * represents *p*<0.01 by student *t*-test. Two to three independent experiments gave similar results.

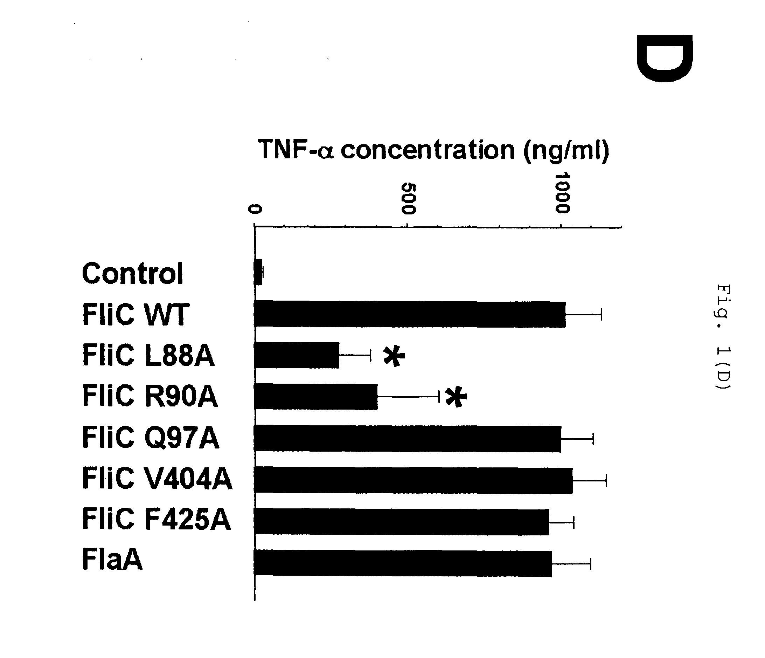

Fig. 1(D) Characterization of flagellin mutants
   Mice were intranasally inoculated with PBS alone (control), or 1 µg of the recombinant protein of FliC WT or mutant (L88A, R90A, Q97A, V404A, or F425A). Four hours after inoculation, the broncho-alveolar lavage fluid was collected and TNF-α concentration was measured by ELISA. Data shows the mean ± SD values. n = 6 mice per group. Mark * represents *p*<0.05 by student *t*-test. Two to three independent experiments gave similar results.
Fig. 1(E) Characterization of flagellin mutants
   Reactivity of anti-FliC WT IgG raised in BALB/c mice with the recombinant protein of FliC WT, L88A, R90A, or FlaA was examined by ELISA. Two to three independent experiments gave similar results.
Fig. 2(A) Immunogenicity of flagellin DNA vaccines
   Cross-reactivity of IgG raised by FliC WT, R90A, or FlaA was examined by immunoblot analysis using flagella prepared from PAO1 or PAK endogenously expressing FliC or FlaA, respectively. For PAO1 blots, proximal bands correspond to the size estimated from the amino acid sequence, while distal bands were estimated to be degraded products.

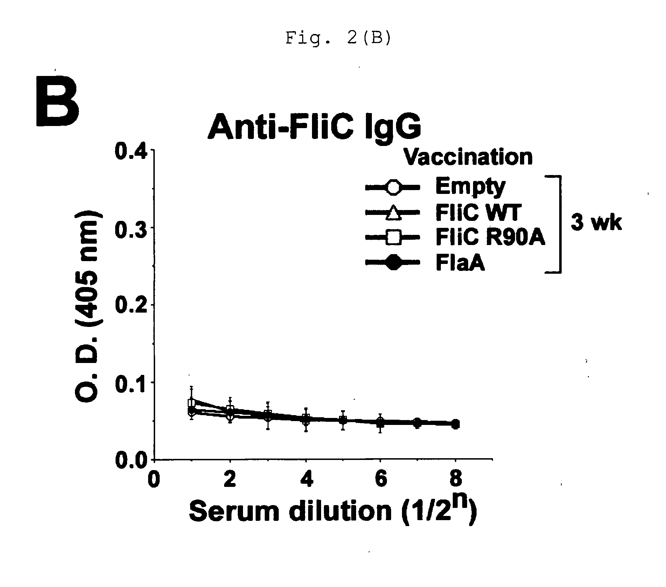

Fig. 2(B) Immunogenicity of flagellin DNA vaccines
   BALB/c mice were immunized twice (at weeks 0 and 3) with either 50 µg of pGACAG (vector), pGACAG-FliC WT, pGACAG-FliC R90A, or pGACAG-FlaA *via* intramuscular electroporation. Blood was collected 3 weeks after the first immunization. Serum IgG titers against FliC (type-B flagella) purified from PAO1 were determined by ELISA. The graph shows the mean ± SD values. n = 8 mice per group. Mark * represents *p*<0.05 by student *t*-test. Three independent experiments gave similar results.

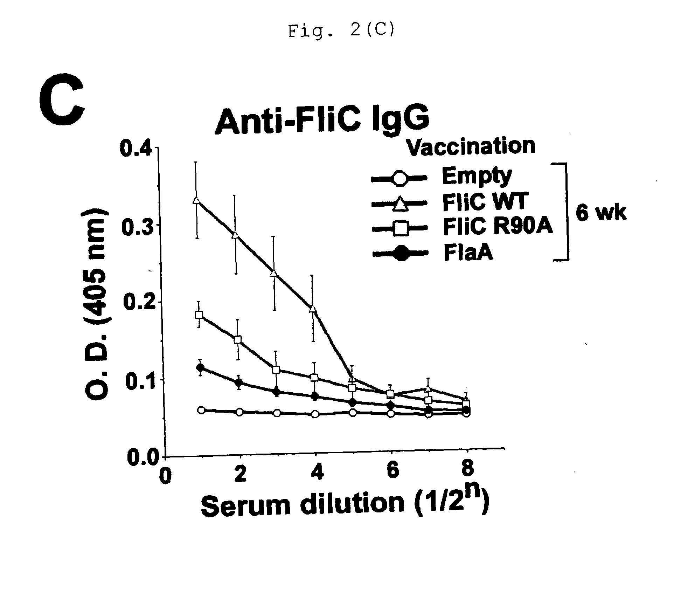

Fig. 2(C) Immunogenicity of flagellin DNA vaccines
   BALB/c mice were immunized twice (at weeks 0 and 3) with either 50 µg of pGACAG (vector), pGACAG-FliC WT, pGACAG-FliC R90A, or pGACAG-FlaA *via* intramuscular electroporation. Blood was collected 6 weeks after the first immunization. Serum IgG titers against FliC (type-B flagella) purified from PAO1 were determined by ELISA. The graph shows the mean ± SD values. n = 8 mice per group. Mark * represents *p*<0.05 by student *t*-test. Three independent experiments gave similar results.

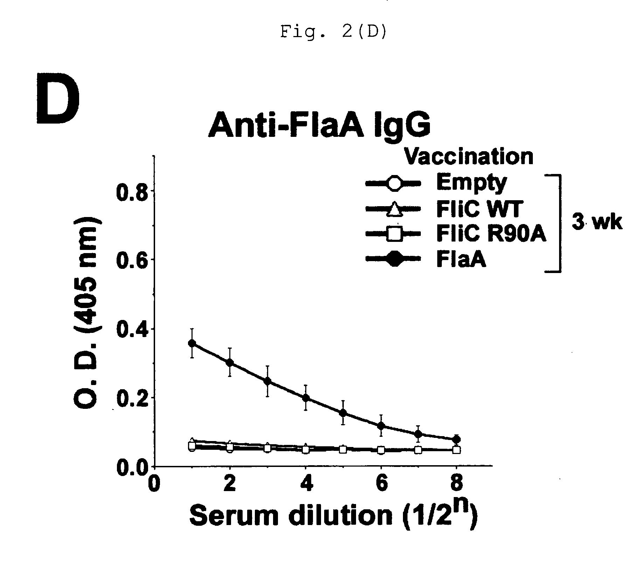

Fig. 2(D) Immunogenicity of flagellin DNA vaccines
   BALB/c mice were immunized twice (at weeks 0 and 3) with either 50 µg of pGACAG (vector), pGACAG-FliC WT, pGACAG-FliC R90A, or pGACAG-FlaA *via* intramuscular electroporation. Blood was collected 3 weeks after the first immunization. Serum IgG titers against FlaA (type-A flagella) purified from PAK were determined by ELISA. The graph shows the mean ± SD values. n = 8 mice per group. Mark * representsp<0.05 by student *t*-test. Three independent experiments gave similar results.

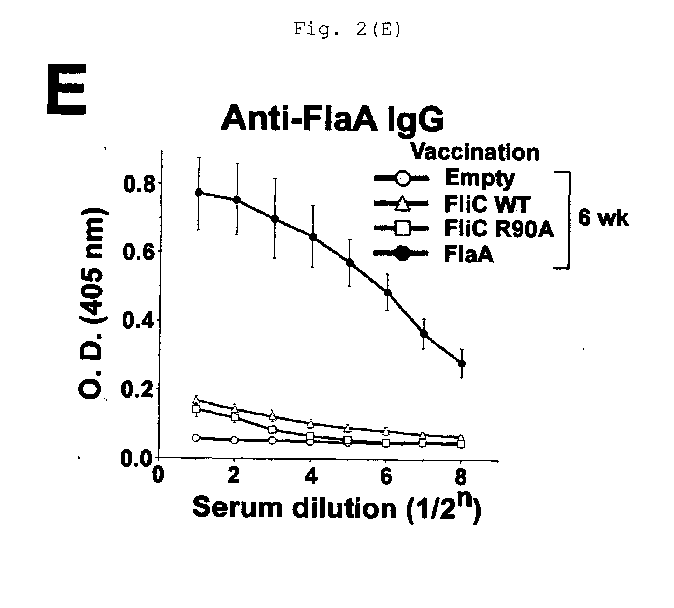

Fig. 2(E) Immunogenicity of flagellin DNA vaccines
   BALB/c mice were immunized twice (at weeks 0 and 3) with either 50 µg of pGACAG (vector), pGACAG-FliC WT, pGACAG-FliC R90A, or pGACAG-FlaA *via* intramuscular electroporation. Blood was collected 6 weeks after the first immunization. Serum IgG titers against FlaA (type-A flagella) purified from PAK were determined by ELISA. The graph shows the mean ± SD values. n = 8 mice per group. Mark * presents p<0.05 by student *t*-test. Three independent experiments gave similar results.

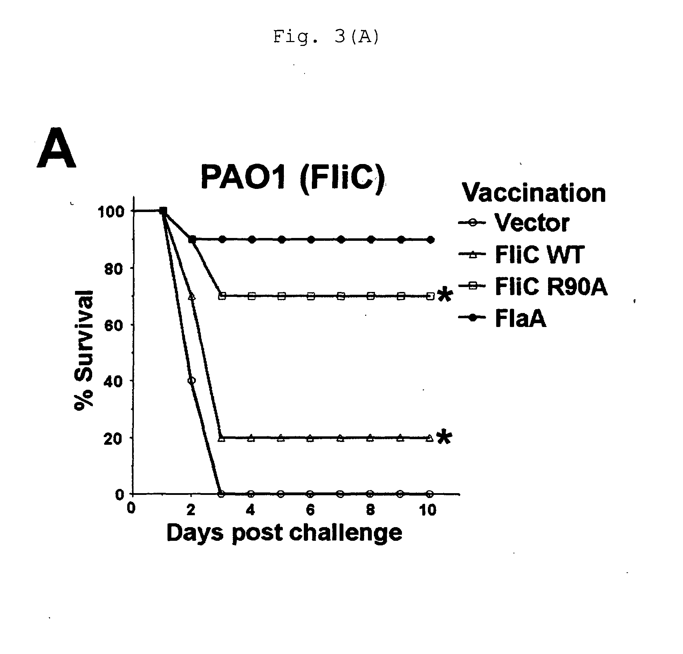

Fig. 3 (A) Protective potential of flagellin DNA vaccines
   Mice were immunized as described in Fig. 2 legend. Survival rate was monitored following intranasal challenge with 2 LD₅₀ doses of PAO1. Three independent experiments gave similar results. Mark * represents *p* < 0.05 by Mantel-Cox Log rank test.

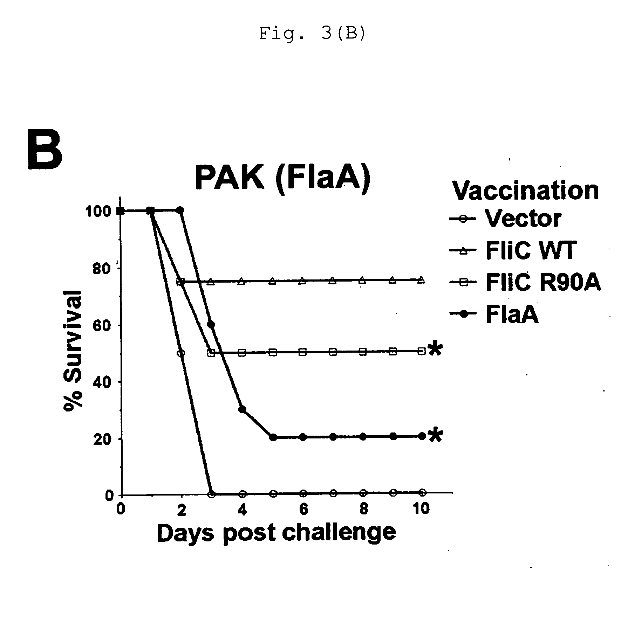

Fig. 3(B) Protective potential of flagellin DNA vaccines
   Mice were immunized as described in Fig. 2 legend. Survival rate was monitored following intranasal challenge with 2 LD₅₀ doses of PAK. Three independent experiments gave similar results. Mark * represents *p* < 0.05 by Mantel-Cox Log rank test.

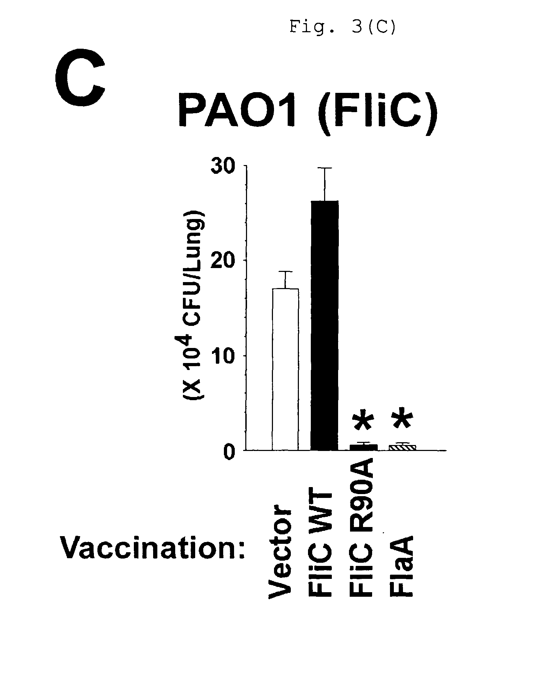

Fig. 3(C) Protective potential of flagellin DNA vaccines
   Mice were immunized as described in Fig. 2 legend. Two weeks after final immunization, mice were challenged intranasally with 5x10⁵ CFU of PAO1. Twenty-four hours post infection, mice were sacrificed and the lung was removed. Viable cell count in the lung homogenate was determined. The graph shows mean ± SD values. n = 5 mice per group. Similar results were obtained in three independent experiments. Mark * represents *p*<0.0001 by student *t*-test.

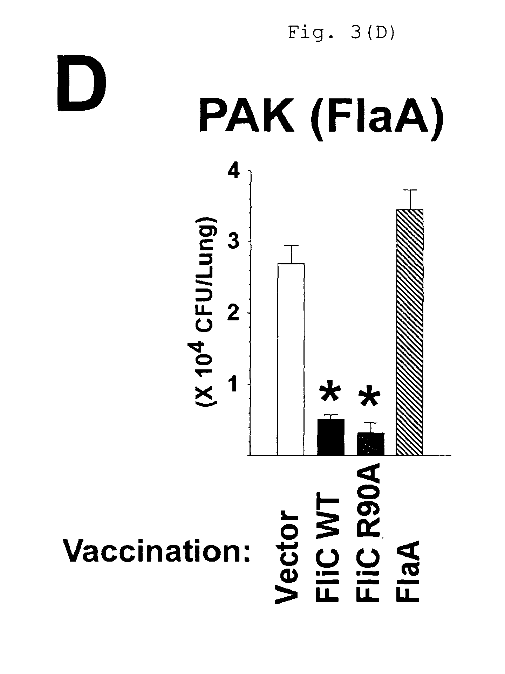

Fig. 3 (D) Protective potential of flagellin DNA vaccines
   Mice were immunized as described in Fig. 2 legend. Two weeks after final immunization, mice were challenged intranasally with 5 x10⁵ CFU of PAK. Twenty-four hours post infection, mice were sacrificed and the lung was removed. Viable cell count in the lung homogenate was determined. The graph shows mean ± SD values. n = 5 mice per group. Similar results were obtained in three independent experiments. Mark * represents *p*<0.0001 by student *t*-test.

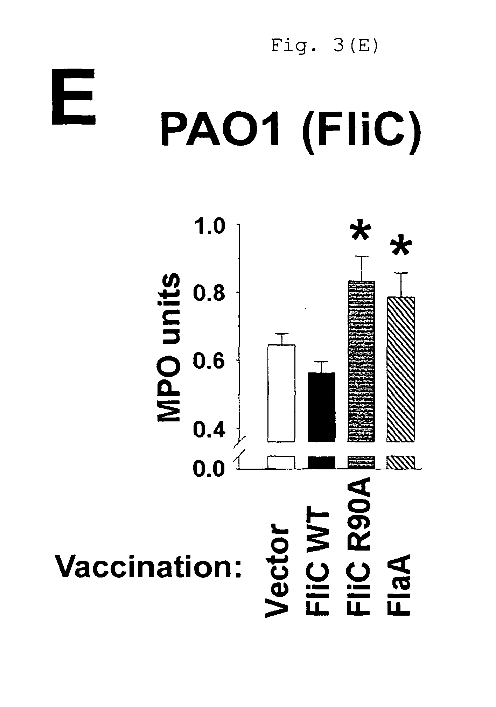

Fig. 3(E) Protective potential of flagellin DNA vaccines
   Mice were immunized as described in Fig. 2 legend. Two weeks after final immunization, mice were challenged intranasally with 5x10⁵ CFU of PAO1. Twenty-four hours post infection, mice were sacrificed and the lung was removed. MPO activity in the lung homogenate was determined. The graph shows mean ± SD values. n = 5 mice per group. Mark * represents *p*<0.05 by student *t*-test. Similar results were obtained in three independent experiments.
Fig. 4(A)
   Type specific anti-flagellin antibody inhibits TLR5 activation by homologous type flagellin, but does not inhibit the activation by heterologous type flagellin.
   HEK293 cells were transiently transfected as described in Fig. 1 legend. The cells were stimulated with different concentrations of recombinant FliC in the presence of either 10 µg/ml of control IgG, anti-FliC WT IgG, anti-FliC R90A IgG or anti-FlaA IgGs and then luciferase assay was performed. Three independent experiments gave similar results. Mark * represents *p*<0.01 by student *t*-test.
Fig. 4(B)
   Type specific anti-flagellin antibody inhibits TLR5 activation by homologous type flagellin, but does not inhibit the activation by heterologous type flagellin.
   HEK293 cells were transiently transfected as described in Fig. 1 legend. The cells were stimulated with different concentrations of recombinant FliC in the presence of either 10 µg/ml of control IgG, anti-FliC WT IgG, anti-FliC R90A IgG or anti-FlaA IgGs and then luciferase assay was performed. Three independent experiments gave similar results. Mark * represents *p*<0.01 by student *t*-test.
Fig. 4(C)
   Type specific anti-flagellin antibody inhibits TLR5 activation by homologous type flagellin, but does not inhibit the activation by heterologous type flagellin.
   HEK293 cells were transiently transfected as described in Fig. 1 legend. The cells were stimulated with different concentrations of recombinant FlaA in the presence of either 10 µg/ml of control IgG, anti-FliC WT IgG, anti-FliC R90A IgG or anti-FlaA IgGs and then luciferase assay was performed. Three independent experiments gave similar results. Mark * represents *p*<0.01 by student *t*-test.
Fig. 4(D)
   Type specific anti-flagellin antibody inhibits TLR5 activation by homologous type flagellin, but does not inhibit the activation by heterologous type flagellin.
   HEK293 cells were transiently transfected as described in Fig. 1 legend. The cells were stimulated with different concentrations of recombinant FlaA in the presence of either 10 µg/ml of control IgG, anti-FliC WT IgG, anti-FliC R90A IgG or anti-FlaA IgC; and then luciferase assay was performed. Three independent experiments gave similar results. Mark * represents *p*<0.01 by student *t*-test.
Fig. 4(E)
   Type specific anti-flagellin antibody inhibits TLR5 activation by homologous type flagellin, but does not inhibit the activation by heterologous type flagellin.
   One microgram of rFliC was pre-incubated with 20 µg of anti-FliC WT IgG or anti-R90A IgG at 37°C for 30 min. BALB/c mice were inoculated intranasally with this mixture. Four hours after inoculation, mice were challenged with 2 LD₅₀ doses of PAO1 and survival rate was monitored for the subsequent 10 days. n = 10 mice per group. Mark * represents *p*<0.01 by Mantel-Cox Log rank test. Similar results were obtained in three independent experiments.

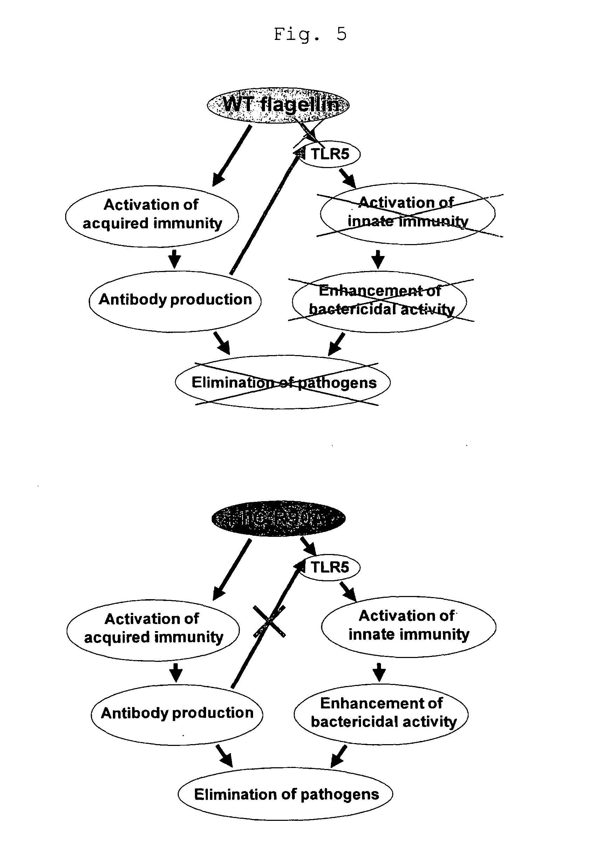

Fig. 5 shows a mechanism by which flagellated pathogens such as *P. aeruginosa* enhance hosts' antibody response to thereby escape from hosts' innate bactericidal activity. While TLR5 enhances the immunogenicity of flagellin via antigenicity reinforcement action, antibody response to TLR5 activation domain inhibits hosts' innate protective immune response to flagellated pathogens such as *P. aeruginosa.* Therefore, inoculation of a wild-type flagellin as a vaccine is not effective for elimination of bacteria expressing homologous flagellin. On the other hand, FliC R90A induces the production of neutralizing antibody to TLR5 only at a minimum level, and there is antigenic homology between *P. aeruginosa* flagellins. Thus, inoculation of FliC R90A as a vaccine will has protective potential against both homologous flagellin-expressing strains and heterologous flagellin-expressing strains.

### BEST MODE FOR CARRYING OUT THE INVENTION

Hereinbelow, the embodiment of the present invention will be described in more detail.

### 1. Flagellin Mutant

The present invention provides a flagellin mutant into which a mutation has been introduced at least at one site in the Toll-like receptor 5 (TLR5) activation domain of a corresponding wild-type flagellin to thereby attenuate the ability to activate TLR5. The flagellin mutant of the present invention may be one which is inert to TLR5 but retains the antigenicity of flagellin.

Flagella, which are organelles essential for the motility of bacteria, are found in a great number of bacterial species. With respect to flagella of pathogens, the flagella of *P. aeruginosa,* Salmonella *(Salmonella typhi), Vibrio cholerae,* Listeria, etc. have been studied well. Bacterial flagella are built up by polymerization of a protein called flagellin. Type A flagella expressed in *Pseudomonas* bacteria are composed of flagellin FlaA and Type B flagella are composed of flagellin FliC. There is about 60% homology between FlaA and FliC, and their antigenicities are partially overlapped with each other. The specific immune response induced by immunizing one of these flagellins also responses to the other flagellin. Although FlaA and FliC alone are known in *P. aeruginosa,* other bacteria are expressing their inherent flagellins.

Toll-like receptor (TLR) is possessed by mammals and is type I membrane protein mainly expressed on cell membranes. It is believed that the leucine rich repeat in the extracellular domain of TLR is critical to its interaction with flagellin and that the Toll/IL-1R domain in the intracellular domain plays an important role in signal transduction. Since TLR5-deficient mice have attenuated response to flagellin, it was revealed that TLR5 is a receptor for flagellin.

From the conformation of flagellin, it has been found that flagellin has D1, D2 and D3 domains. D1 domain is composed of an α-helix structure located near the NH₂- and COOH-termini and is believed to be critical to activation of TLR5. From experiments using mutants, it was believed with respect to *P. aeruginosa* FliC that a region spanning from amino acid position 71 to position 97 is critical for the structure of TLR5 activation domain.

It is known that when TLR5 is activated by the action of flagellin (its ligand), TLR5 activates NF-κB through intracellular adaptor molecules MyD88, IRAK1 and TRAF6. When human kidney-derived fibroblast cell line HEK293 is transfected with TLR5 expression plasmid and NF-κ dependent luciferase expression plasmid and challenged with flagellin, intracellular luciferase activity increases in a flagellin concentration dependent manner. By using this experimental system, it becomes possible to measure the ability to activate TLR5.

The wild-type flagellin used in the present invention may be derived from a flagellated pathogen, preferably *P. aeruginosa.* More preferably, the wild-type flagellin is *P. aeruginosa-derived* FlaA and FliC, still more preferably *P. aeruginosa-derived* FliC.

The amino acid sequence and the nucleotide sequence of *P. aeruginosa* (PAO1)-derived wild-type flagellin FliC are shown in SEQ ID NOS: 1 and 2, respectively

Generally, proteins take various conformations. Therefore, it is not possible to determine the TLR5 activation domain of flagellin unambiguously. However, for example, it is believed that the region spanning from position 71 to position 97 of the amino acid sequence shown in SEQ ID NO: 2 is the TLR5 activation domain of wild-type flagellin FliC.

The flagellin mutant of the present invention may be a flagellin mutant having the amino acid sequence as shown in SEQ ID NO: 2 in which arginine at position 90 is substituted with another amino acid. The "another amino acid" may be an amino acid which does not destroy the conformation of flagellin. For example, alanine or glycine may be given.

Specific examples of the flagellin mutant of the present invention include the following proteins (a), (b) and (c).
(a) a flagellin mutant protein having the amino acid sequence as shown in SEQ ID NO: 2 wherein arginine at position 90 is substituted with alanine;
(b) a flagellin mutant protein which has the amino acid sequence of the protein of (a) wherein one or more amino acids (preferably 2 to 20 amino acids, more preferably 2 to 10 amino acids) other than alanine at position 90 are deleted, substituted or added, and whose Toll-like receptor 5 activation ability is attenuated compared to the corresponding ability of wild-type flagellin FliC having the amino acid sequence as shown in SEQ ID NO: 2; and
(c) a flagellin mutant protein which is encoded by a DNA that hybridizes to a complementary DNA to a DNA encoding the protein of (a) under stringent conditions, and whose Toll-like receptor 5 activation ability is attenuated compared to the corresponding ability of wild-type flagellin FliC having the amino acid sequence as shown in SEQ ID NO: 2.

With respect to the protein of (c) above, the "DNA that hybridizes to a complementary DNA to a DNA encoding the protein of (a) under stringent conditions" may be at least 80% (preferably at least 95%, more preferably at least 98%) identical with the whole or a part of the complementary DNA to the DNA encoding the protein of (a). The hybridization is performed under stringent conditions. The stability of nucleic acid duplex or hybrid duplex is represented by melting temperature (Tm) (the temperature at which a probe is dissociated from its target DNA). This melting temperature is used for defining stringent conditions. Suppose that 1% mismatch lowers Tm by 1°C, the temperature at the time of final washing in hybridization reaction should be set low. For example, when a DNA sequence having 95% or more identity with a probe is sought for, the final washing temperature should be lowered by 5°C. Actually, Tm will change by 0.5-1.5°C by 1% mismatch. Specific examples of stringent conditions include, but are not limited to, hybridization in 5x SSC/5x Denhardt's solution/1.0% SDS at 68°C and washing in 0.2x SSC/0.1% SDS at room temperature. One example of medium stringent conditions is washing in 3x SSC at 42°C. Salt concentrations and temperatures may be changed to achieve an optimum level of identity between probe and target nucleic acid. For a further guide to such conditions, see Sambrook et al., 1989, Molecular Cloning, A Laboratory Manual, Cold Spring Harbor Press, N.Y; and Ausubel et al. (eds.), 1995, Current Protocols in Molecular Biology, (John Wiley & Sons. N.Y) at Unit 2.10.

The amino acid sequence of the protein of (a) is shown in SEQ ID NO: 4. As one example of the DNA encoding the protein of (a), a DNA having the nucleotide sequence as shown in SEQ ID NO: 3 may be given.

The flagellin mutant of the present invention may be prepared by known methods. For example, a DNA encoding a flagellin mutant may be obtained as described in 2 below; after integration of the DNA into an appropriate expression vector, the vector may be introduced into an appropriate host; then, the flagellin mutant of interest may be produced as a recombinant protein (see, for example, Current Protocols Compact Edition, Molecular Biology Experimental Protocols I, II, III, translated by Kaoru Saigo and Yumiko Sano, published by Matuzen; the original of the above translation: Ausubel, F.M. et al, Short Protocols in Molecular Biology, Third Edition, John Wiley & Sons, Inc., New York).

Alternatively, the flagellin mutant of the present invention may be prepared by known peptide synthesis methods.

### 2. Isolated DNA Encoding the Flagellin Mutant

An isolated DNA encoding the flagellin mutant of the present invention may be any DNA as long as it comprises a nucleotide sequence encoding the flagellin mutant of the present invention. Examples of the DNA encoding the flagellin mutant of the present invention include, but are not limited to, a DNA having the nucleotide sequence as shown in SEQ ID NO: 3; and a DNA which hybridizes under stringent conditions to a complementary DNA to a DNA encoding a flagellin mutant having the amino acid sequence as shown in SEQ ID NO: 4 (the amino acid sequence as shown in SEQ ID NO: 2 wherein arginine at position 90 is substituted with alanine) (e.g., DNA having the nucleotide sequence as shown in SEQ ID NO: 3) and encodes a flagellin mutant protein whose ability to activate TLR5 is attenuated compared to the corresponding ability of wild-type flagellin FliC having the amino acid sequence as shown in SEQ ID NO: 2.

The DNA which hybridizes under stringent conditions to a complementary DNA to a DNA encoding a flagellin mutant having the amino acid sequence as shown in SEQ ID NO: 4 may be at least 80% (preferably at least 95%, more preferably at least 98%) identical with the whole or a part of the above-mentioned complementary DNA. The hybridization is performed under stringent conditions. The stability of nucleic acid duplex or hybrid duplex is represented by melting temperature (Tm) (the temperature at which a probe is dissociated from its target DNA). This melting temperature is used for defining stringent conditions. Suppose that 1% mismatch lowers Tm by 1°C, the temperature at the time of final washing in hybridization reaction should be set low. For example, when a DNA sequence having 95% or more identity with a probe is sought for, the final washing temperature should be lowered by 5°C. Actually, Tm will change by 0.5-1.5°C by 1% mismatch. Specific examples of stringent conditions include, but are not limited to, hybridization in 5x SSC/5x Denhardt's solution/1.0% SDS at 68°C and washing in 0.2x SSC/0.1% SDS at room temperature. One example of medium stringent conditions is washing in 3x SSC at 42°C. Salt concentrations and temperatures may be changed to achieve an optimum level of identity between probe and target nucleic acid. For a further guide to such conditions, see Sambrook et al., 1989, Molecular Cloning, A Laboratory Manual, Cold Spring Harbor Press, N.Y; and Ausubel et al. (eds.), 1995, Current Protocols in Molecular Biology, (John Wiley & Sons. N.Y) at Unit 2.10.

As one example of the DNA encoding a flagellin mutant having the amino acid sequence as shown in SEQ ID NO: 4, a DNA having the nucleotide sequence as shown in SEQ ID NO: 3 may be given.

The isolated DNA encoding the flagellin mutant of the present invention may be prepared, for example, as described below.

Briefly, genomic DNA is extracted from *P. aeruginosa* followed by amplification of a flagellin-encoding region (487 residues) by PCR The resultant PCR product is a DNA encoding a wild-type flagellin. The amino acid sequence of wild-type flagellin FliC and one example of the nucleotide sequence of a DNA encoding FliC are shown in SEQ ID NOS: 2 and 1, respectively.

A DNA encoding a flagellin mutant may be prepared by introducing a mutation into a desired site within the flagellin-encoding region (487 residues) by site-directed mutagenesis. The mutated flagellin-encoding region (487 residues) is amplified by PCR. The resultant PCR product is a DNA encoding the flagellin mutant.

One example of the nucleotide sequence of a DNA encoding a flagellin mutant consisting of the amino acid sequence as shown in SEQ ID NO: 2 in which arginine at position 90 is substituted with alanine is shown in SEQ ID NO: 3.

SEQ ID NOS: 5 and 6 show the nucleotide sequence and the amino acid sequence of *P. aeruginosa* (PAK)-derived wild-type flagellin FlaA, respectively. The homology between the amino acid sequences of wild-type flagellins FliC and FlaA is about 60%. It is believed that arginine at position 90 of the amino acid sequence as shown in SEQ ID NO: 2 corresponds to arginine at position 90 of the amino acid sequence as shown in SEQ ID NO: 6. Therefore, those flagellin mutants having the amino acid sequence as shown in SEQ ID NO: 6 wherein arginine at position 90 is substituted with another amino acid (e.g., alanine, glycine, etc.) are also included in the present invention.

### 3. Recombinant Vector

A recombinant vector comprising a DNA encoding the flagellin mutant of the present invention may be obtained by inserting the DNA into an appropriate expression vector by known methods (e.g., methods described in Molecular Cloning 2nd Edition, J. Sambrook et al., Cold Spring Harbor Lab. Press, 1989).

As the expression vector, *Escherichia coli*-derived plasmids (e.g., pBR322, pBR325, pUC12, pUC13 or pGACAG); *Bacillus subtilis-derived* plasmids (e.g., pUB110, pTP5 or pC194), yeast-derived plasmids (e.g., pSH19 or pSH15); bacteriophages such as λ phage; animal viruses such as retrovirus, adenovirus or vaccinia virus; or insect pathogen viruses such as baculovirus may be used.

The expression vector may also comprise promoters, enhancers, splicing signals, poly-A addition signals, selection markers, SV40 replication origins, and so forth.

The expression vector may be a fusion protein expression vector. Various fusion protein expression vectors are commercially available. For example, pGEX series (Amersham Pharmacia Biotech), pET CBD Fusion System 34b-38b (Novagen), pET Dsb Fusion Systems 39b and 40b (Novagen) and pET GST Fusion System 41 and 42 (Novagen) may be enumerated.

When a recombinant vector is to be used as a gene vaccine (plasmid vaccine or virus vector vaccine), a gene of interest may be introduced into pGACAG plasmid vector that has been already developed for gene vaccines to thereby prepare a plasmid vaccine.

### 4. Transformant

A transformant may be obtained by introducing into a host a recombinant vector comprising a DNA encoding the flagellin mutant of the present invention.

Examples of the host include, but are not limited to, bacterial cells (such as *Escherilia* bacteria, *Bacillus* bacteria or *Bacillus subtilis),* fungal cells (such as yeast or *Aspergillus),* insect cells (such as S2 cells or Sf cells), animal cells (such as CHO cells, COS cells, HeLa cells, C127 cells, 3T3 cells, BHK cells or HEK 293 cells) and plant cells.

Introduction of a recombinant vector into a host may be performed by the methods described in Molecular Cloning 2nd Edition, J. Sambrook et al., Cold Spring Harbor Lab. Press, 1989 (e.g., the calcium phosphate method, the DEAE-dextran method, transfection, microinjection, lipofection, electroporation, transduction, scrape loading, the shotgun method, etc.) or by infection.

The transformant may be cultured in a medium to collect the flagellin mutant from the resultant culture. When the flagellin mutant is secreted into the medium, the medium may be recovered, followed by isolation and purification of the flagellin mutant from the medium. When the flagellin mutant is produced within the transformed cells, the cells may be lysed, followed by isolation and purification of the flagellin mutant from the cell lysate.

When the flagellin mutant is expressed in the form of a fusion protein with another protein (functioning as a tag), the fusion protein may be isolated/purified and then treated with FactorXa or enterokinase to thereby cut off the other protein. Thus, the flagellin mutant of interest may be obtained.

Isolation and purification of the flagellin mutant may be performed by known methods. Known isolation/purification methods which may be used in the present invention include, but are not limited to, methods using difference in solubility (such as salting-out or solvent precipitation); methods using difference in molecular weight (such as dialysis, ultrafiltration, gel filtration or SDS-polyacrylamide gel electrophoresis); methods using difference in electric charge (such as ion exchange chromatography); methods using specific affinity (such as affinity chromatography); methods using difference in hydrophobicity (such as reversed phase high performance liquid chromatography); and methods using difference in isoelectric point (such as isoelectric focusing).

### 5. Vaccine

The present invention also provides a vaccine comprising the flagellin mutant, a DNA encoding the flagellin mutant, or a vector comprising the DNA. The vaccine of the present invention is a vaccine against flagellated pathogens and is especially effective against *P. aeruginosa.* The vaccine of the present invention may be used, for example, for protection against cystic fibrosis and/or protection of medically compromised patients where *P. aeruginosa* causes a serious problem.

The flagellin mutant, the DNA encoding the flagellin mutant, or the vector comprising the DNA are explained above.

The flagellin mutant, the DNA encoding the flagellin mutant, or the vector comprising the DNA may be dissolved in a buffer (such as PBS), physiological saline, sterilized water, or the like; the resultant solution may be injected into subjects, if necessary, after filter-sterilization. Additives such as inactivators, preservatives, adjuvants or emulsifiers may be added to the above solution. The flagellin mutant may be administered intravenously, intramuscularly, intraperitoneally, subcutaneously, intradermally, or the like. Alternatively, the flagellin mutant may be administered intranasally or orally.

The dose of the flagellin mutant, the DNA encoding the flagellin mutant, or the vector comprising the DNA, and the number of times and frequency of administration may vary depending on the condition, age and body weight of the subject, the administration route, the dosage form, etc. For example, usually, the flagellin mutant of the present invention may be administered at 0.01-1 mg/kg body weight, preferably 0.01-0.1 mg/kg body weight, per adult at least once, at a frequency with which the desired effect is retained. When a DNA encoding the flagellin mutant or a vector comprising the DNA is administered, the DNA or the vector may be administered, for example, usually at 1-100 mg/adult, preferably 1-10 mg/adult, at least once, at a frequency with which the desired effect is retained. When the vector is a virus, the virus may be administered at 10⁹-10¹² viral particles/adult, preferably 10¹¹-10¹² viral particles/adult, at least once, at a frequency with which the desired effect is retained.

Plasmid vaccines may be administered after removal of endotoxin.

Various methods for enhancing vaccine effect may also be used jointly; for example, a method in which a vaccine is introduced into cells by electroporation after intramuscular administration; or a method in which a vaccine and a transfection enhancer (such as liposome) are made into a complex and then administered.

### 6. Antibody

The present invention also provides an antibody induced against the flagellin mutant of the present invention. The antibody of the present invention is capable of responding to a wide variety of *P. aeruginosa* effectively.

The antibody of the present invention may be obtained by administering to an animal an antigen or antigenic epitope, or a DNA encoding the same according to conventional protocols.

The antibody of the present invention may be any one of the following: polyclonal antibody, monoclonal antibody, chimeric antibody, single chain antibody and humanized antibody.

Polyclonal antibodies may be prepared by a known method, optionally with necessary modifications. For example, an immunizing antigen (protein antigen or plasmid DNA expressing the same) may be administered to an animal (immunization), and then a material containing antibodies to the protein may be collected from the immunized animal, and the antibodies may be isolated/purified to thereby prepare the antibody of interest. At the time of antigen administration, complete Freund's adjuvant or incomplete Freund's may also be administered to enhance antibody production potential. Usually, the antigen may be administered at intervals of about 2 to 6 weeks in the total of about 2 to 10 times. Polyclonal antibodies may be collected from the blood, abdominal dropsy or the like of immunized animal. Preferably, they are collected from the blood. Polyclonal antibodies may be isolated and purified according to the methods used for isolation and purification of immunoglobulins (e.g., salting-out, alcohol precipitation, isoelectric precipitation, electrophoresis, absorption/desorption using ion exchangers, ultracentrifugation, gel filtration, or a specific purification method in which antibody alone is collected with an antigen-bound solid phase of an active adsorbent such as protein A or protein G and then dissociated to obtain the antibody). As a polyclonal antibody, IgG fraction purified from serum is preferable.

Monoclonal antibodies may be prepared by the hybridoma method of G Koehler and C. Milstein described in Nature (1975) 256: 495, Science (1980) 208: 692-. Briefly, after immunization of an animal, antibody-producing cells are isolated from the spleen of the immunized animal. By fusing these cells with myeloma cells, monoclonal antibody-producing cells are prepared. Cell clones may be isolated which produce monoclonal antibodies that respond to various *P. aeruginosa* strains-derived flagellins and protect against a wide spectra of such *P. aeruginosa* strains. The thus obtained cell clone may be cultured, and a monoclonal antibody of interest may be obtained from the resultant culture. Monoclonal antibodies may be purified by the above-listed methods for isolation and purification of immunoglobulins.

A method for preparing a single-chain antibody is disclosed in US Patent No. 4,946,778.

A method for preparing a humanized antibody is disclosed in Biotechnology 10, 1121-, 1992; and Biotechnology 10, 169-, 1992.

The antibody of the present invention may be used for treatment of infections with flagellated bacteria, in particular *P. aeruginosa.* The advantage of this medicine is that its effect does not need to be waited until host's immune system has been activated and reached a sufficiently high protection level; the effect can be expected immediately. In particular, when an infection has been established or a disease has been established, the antibody of the present invention is capable of immediately recovering patients from the pathogen or toxin produced by the pathogen. For example, the antibody of the present invention may be dissolved in a buffer (such as PBS), physiological saline, sterilized water, or the like; the resultant solution may be injected into subjects, if necessary, after filter-sterilization. Additives such as coloring agents, emulsifiers, suspending agents, surfactants, dissolution aids, stabilizers, preservatives, anti-oxidants, buffers or isotonizing agents may be added to the above solution. This solution may be administered intravenously, intramuscularly, intraperitoneally, subcutaneously, intradermally, or the like. Alternatively, the solution may be administered intranasally or orally.

The dose of the antibody of the present invention and the number of times and the frequency of its administration may vary depending on the condition, age and body weight of the subject, the administration route, the dosage form, etc. For example, usually, the antibody of the present invention may be administered at 1,000-10,000 mg/kg body weight, preferably 2,000-5,000 mg/kg body weight, per adult at least once, at a frequency with which the desired effect is retained.

### EXAMPLES

Hereinbelow, the present invention will be described in detail based on the following Example. However, the present invention is not limited to this Example.

### [EXAMPLE 1] DNA vaccine encoding a novel flagellin mutant confers protective potential against P. aeruginosa without inducing antibody that inhibit TLR5-mediated host's innate immune response.

### ABSTRACT

Flagellin is a key component of the flagella of many pathogens, and raises a distinct pattern of host immune responses through TLR5. DNA vaccines were generated which encodes the two major types of flagellin (FliC and FlaA) expressed by various strains of *P*. *aeruginosa,* respectively. Unexpectedly, each DNA vaccine induced stronger protection against bacteria expressing the heterologous type flagellin to the integrated flagellin than bacteria expressing homologous type flagellin. This phenomenon seems to be associated with the ability of antibodies raised against homologous flagellin to inhibit the activation of TLR5-mediated innate immune response, thereby reducing the host's protective potential against to infection. To circumvent this limitation and generate a broadly cross-protective DNA vaccine, site-directed flagellin mutants were generated. One of such mutants, FliC R90A, showed >100-fold lower potential for TLR5 activation but retained an antigenic epitope as a flagellin antigen. Vaccination with FliC R90A elicited optimal protective immune responses against both FliC- and FlaA-expressing *P. aeruginosa* strains. Thus, these results suggested that neutralizing antibody to the TLR5 activation domain of flagellin may be a critical factor in the construction of vaccines against flagellated pathogens. These observations also raise a possibility that flagellated bacteria have evolved so that they facilitate host's antibody responses to autologous component, in order to evade from host's innate bactericidal activities.

### INTRODUCTION

Flagella are conserved organelles that confer motility to diverse bacterial species, including *P. aeruginosa.* Reduction in flagellar activity restricts motility, and eventually limits organisms' ability to invade/colonize in hosts (2). In view of host's immunity against bacteria, flagella are highly immunogenic and non-flagellated bacteria are difficult to eliminate from living bodies under certain settings (2). In this context, it is reported that anti-flagellar antibodies improve the survival of mice challenged with *P. aeruginosa* (3). Yet flagellin, a key protein component of flagella, can trigger host's innate immune response by interacting with TLR5 (4). It is reported that activation of TLR5 in epithelial cells, macrophages or dendritic cells initiates a signaling cascade mediated by myeloid differentiation factor 88 adaptor molecule, IL-IR-associated kinase, TNFR-associated factor 6 and IκB kinase to induce cytokine production including TNF-α, IL-6, IL-10, IL-12, and IFN-γ; as a result, vaccine efficiency against infection with organisms such as *Vibrio bulnificus* is enhanced (5-12). These reports suggest that a flagellin-specific vaccine may induce a strong protective immune response by stimulating both host's innate and acquired immunity.

In order to examine the efficacy of flagellin-specific vaccines, a series of DNA vaccines encoding 'A' (FlaA) and 'B' (FliC) type flagellins from different serotypes of *P. aeruginosa* (PAK and PAO1) (13) were generated and tested for their protective potential in mouse *P. aeruginosa* acute pneumonia model. As a result, each flagellin DNA vaccine could not protect mice from challenge with bacteria expressing a homologous type flagellin but efficiently protected from those expressing a heterologous type flagellin. Each flagellin vaccine not only raised antibodies cross-reacting with heterologous type flagellin, but also produced IgG fraction neutralizing TLR5 activation domain type-specifically. One of the novel flagellin mutants FliC R90A is attenuated in the ability to activate TLR5 but retains an antigenic epitope. It was demonstrated that FliC R90A confers sufficient immune responses against infection with a plurality of *P. aeruginosa* strains expressing heterologous type flagellin.

### RESULTS and DISCUSSION

### Examination of the Ability of Various Flagellin FliC Mutants to Activate TLR5

To examine the contribution of TLR5 activation by flagellin to its immunogenicity and vaccine efficiency, TLR5 activation-attenuated mutants were designed. Previous studies showed that TLR5 activation by the FliC of *Salmonella typhimurium* or FlaA of *P. aeruginosa* is mediated by a domain composed of the NH₂- and COOH-terminal regions forming several α-helices (14, 15). Based on this information, five *P. aeruginosa* FliC mutants were generated by replacing a single amino acid in each α-helical region with alanine (Fig. 1(A)). Each recombinant protein was purified under endotoxin-free conditions and characterized for its ability to activate TLR5 using HEK293 cells transfected with a mouse or human TLR5 expression plasmid plus an NF-κB-dependent luciferase reporter plasmid. In this system, luciferase activity was not increased by treatment with LPS, peptidoglycan, or recombinant GST that was prepared by same manner as FliC recombinants (data not shown). As shown in Fig. 1(B) and (C), L88A and R90A mutants showed >100-fold lower potential to trigger TLR5-mediated NF-κB activation when compared with wild-type FliC (FliC WT, *p* <0.01). The biological activity of these flagellar proteins was then examined. Intranasal inoculation of recombinant FliC WT, FliC Q97A, FliC V404A, FliC F425A, or FlaA WT strongly up-regulated pulmonary production of TNF-α (Fig. 1(D)). By comparison, the level of TNF-α in broncho-alveolar lavage fluids from mice treated with FliC L88A or FliC R90A was significantly reduced (p<0.05, Fig. 1(D)). To determine whether these mutatnts retained their antigenic epitopes, the ability of IgG antibody raised against FliC to bind to each protein was examined by ELISA. As a result, anti-flagellin antibody bound to FliC WT and R90A equivalently, but not to L88A. Anti-FliC serum also cross-reacted with recombinant FlaA (type A flagellin) with lower affinity (Fig. 1(E)). These findings suggest that FliC R90A retains the antigenic epitope of flagellin but FliC L88A does not. Consequently, the present inventors have succeeded in creating FliC R90A, a mutant which retains the antigenicity as flagellin but its ability to activate TLR5 is significantly attenuated compared with wild-type flagellin.

### Immunogenicity of DNA vaccines expressing FliC WT. R90A, or FlaA

Mice were immunized with DNA vaccines encoding FliC WT, FliC R90A, or FlaA. Sera from these animals were studied for reactivity with flagellar proteins from the PAO1 and PAK strains of *P. aeruginosa* (expressing FliC and FlaA, respectively). Sera from unvaccinated mice did not react with either protein (data not shown). Sera from all of the immunized mice reacted with both types of flagella, suggesting that each vaccine induced antibodies capable of cross-reacting with both types of flagellin (Fig. 2(A)). Antibody titers in individual sera were determined by ELISA (Fig. 2(B)-(E)). After booster immunization, significant production of anti-PAO1 (FliC) and anti-PAK (FlaA) IgG was recognized in all the vaccine administration groups except for the group which received an empty vector (Fig.2(C) and (E)). The FliC R90A DNA vaccine induced weaker antibody responses against both types of flagella compared with those induced by FliC WT vaccine (p <0.05, Fig. 2(C) and (E)). These results suggested either that the mutation introduced into R90A affected its immunoigenicity or that TLR5-mediated activation of the innate immune system contributed to the immunogenicity of FIiC WT vaccine.

### Protective activity of DNA vaccines expressing flagellin

The capacity of each DNA vaccine to protect against *P. aeruginosa* infection was assessed (Fig. 3(A) and (B)). Unexpectedly, mice vaccinated with FlaA were better protected against PAO1 (which expresses FliC) than PAK (which expresses FlaA), whereas mice vaccinated with the FliC DNA vaccine were better protected against PAK than PAO1. These findings suggest that each flagellin vaccine elicited stronger protection against bacteria expressing heterologous type flagellin. It should be noted that FliC R90A DNA vaccine induced strong protection against both strains of *P. aeruginosa* (Fig. 3 (A) and (B)).

The efficacy of each DNA vaccine on host susceptibility to bacterial infection was further evaluated by examining the viable cell count and neutrophil recruitment in the lung following intranasal infection with a sub-lethal dose of *P. aeruginosa.* Consistent with data from the protection studies, mice immunized with DNA vaccines encoding FlaA or FliC R90A efficiently eliminated PAO1, whereas mice immunized with the FliC WT or R90A vaccines efficiently eliminated PAK (Fig. 3(C) and (D)). When neutrophil recruitment was assessed by monitoring myeloperoxidase (MPO) activity, both FliC R90A and FlaA vaccines elicited stronger MPO activity than FliC WT following challenge with PAO1 (Fig. 3(E)). Taken together, these findings revealed an interesting phenomenon that a flagellin-based vaccine induces a neutralizing antibody to TLR5 activation domain to thereby hamper protective potential against bacteria expressing homologous type flagellin, but protective potential against bacteria expressing heterologous type flagellin is not inhibited. These data also indicate that FliC R90A DNA vaccine may provide protection against a wide variety of *P. aeruginosa* strains.

### Anti-flagellin antibody induced by flagellin antigen neutralizes the TLR5 activation domain of flagellin.

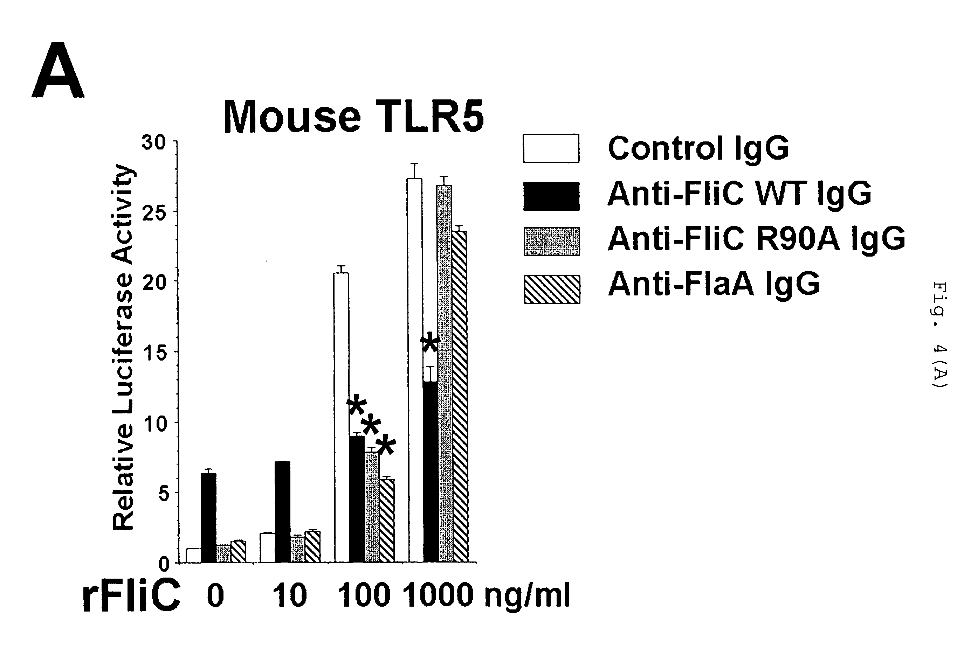

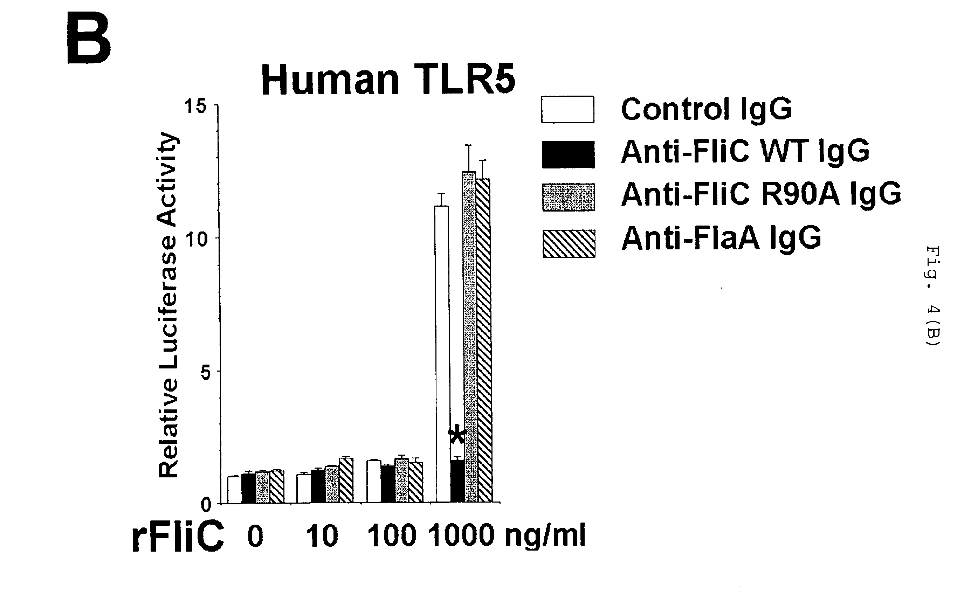

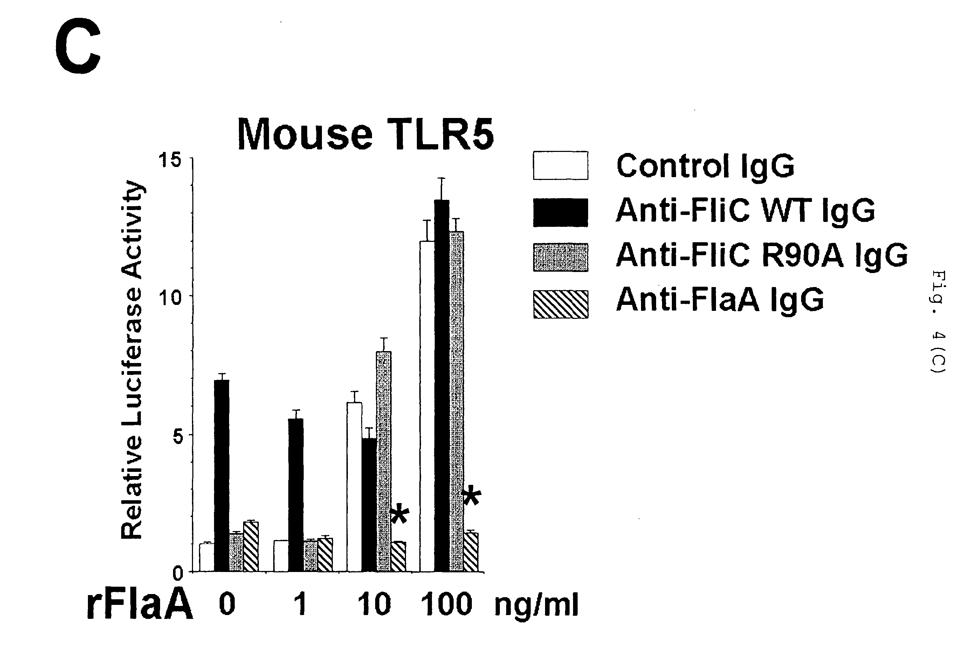

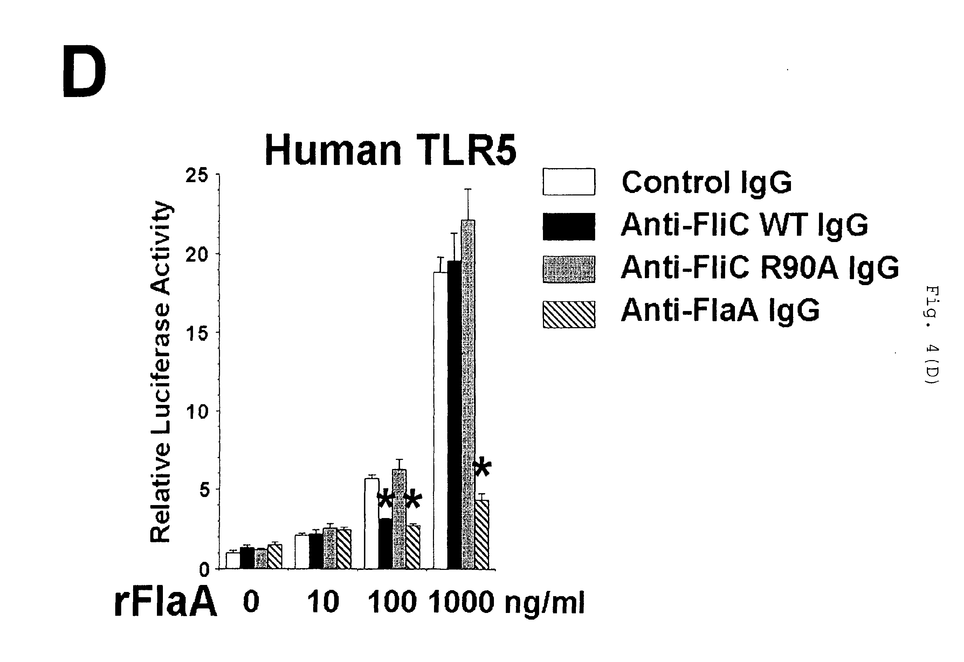

Whether or not IgG fractions induced by flagellin vaccines contain such IgG that neutralizes TLR5 activation by flagellin was examined. Recombinant flagellin proteins dose-dependently stimulated both mouse and human TLR5 in the presence of control IgG (Fig. 4(A)-(D)). IgG raised by FliC WT, FliC R90A or FlaA inhibited mouse TLR5 activation at a lower concentration of rFliC (100 ng/ml, p<0.001); while anti-FliC WT but neither FliC R90A nor FlaA IgG significantly inhibited the activation at a higher concentration of rFliC (1000 ng/ml, *p*<0.001) (Fig. 4(A)). Similar results were observed when human TLR5 activation was examined (Fig. 4(B)). Anti-FlaA IgG specifically inhibited mouse TLR5 activation with rFlaA (*p*<0.01). Both anti-FliC WT and FlaA IgG inhibited human TLR5 activation at a lower concentration of rFlaA (100 ng/ml, *p*<0.01), while anti-FlaA IgG specifically inhibited human TLR5 activation at a higher concentration of rFlaA (1000 ng/ml, *p*<0.01) (Fig. 4(C) and (D)). Combined with data from the protection studies, these results suggest that IgG fraction interacting with the TLR5 activation domain hampers the activation of host's innate immune response by *P. aeruginosa* to thereby enhance host's susceptivility to infection. It was also noted that serum from mice immunized with FliC DNA vaccine was weak but significantly stimulated mouse TLR5. This may reflect epitope mimicry between FliC and the idiotype of anti-FliC antibody, as reported in a previous study (16).

### Anti-flagellin antibody, inhibits activation of host's innate immune response against P. aeruginosa infection.

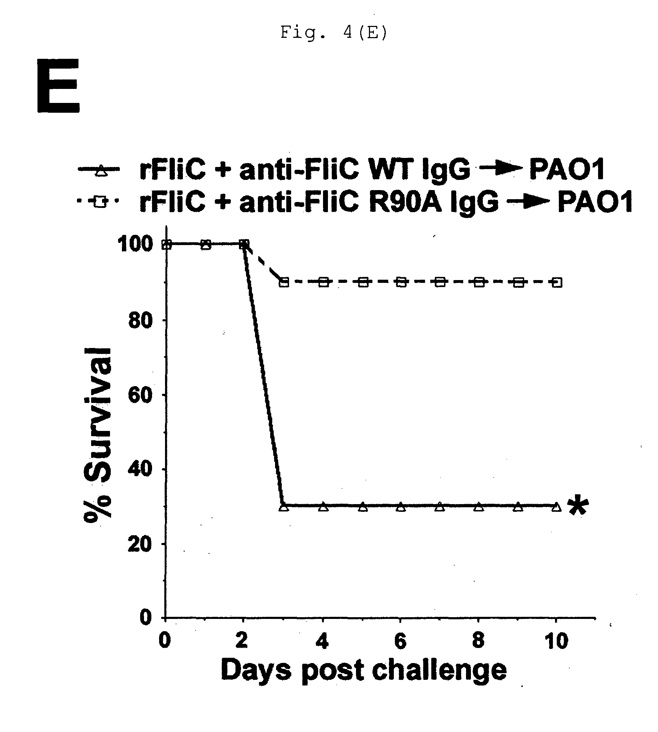

To evaluate the contribution of anti-FliC antibody response to host protection against *P. aeruginosa,* mice were inoculated intranasally with rFliC in the presence of anti-FliC WT or FliC R90A IgG and then challenged with *P. aeruginosa* PAO1 strain (Fig. 4(E)). Pre-treatment with 1 µg of rFliC for 4 hours conferred protection against 2 LD₅₀ doses of intranasal challenge with bacteria (data not shown). This protection was inhibited when rFliC was pre-incubated with anti-FliC WT IgG However, anti-FliC R90A IgG did not inhibit the rFliC-mediated protective effect (*p*<0.01, Fig. 4E). This observation suggests that FliC R90A vaccine does not induce such antibody fraction that inhibits TLR5 activation by flagellin, efficiently elicits host's innate immune response against bacterial infection, and will eliminate bacteria effectively.

Taken together, although flagellin enhances its immunogenicity through TLR5-mediated activation of host's innate immune response (5, 12), induced antibody against TLR5 activation domain hampers host's protective innate immune responses against flagellated pathogens such as *P. aeruginosa.* Indeed, vaccination with WT flagellin is ineffective for elimination of bacteria expressing homologous type flagellin. It was demonstrated that FliC R90A raises a minimal level of IgG that inhibits TLR5 activation and that due to antigenic homology among *P. aeruginosa* flagellins, vaccination with FliC R90A efficiently induces protective immune response against both strains expressing homologous and heterologous type flagellins. This finding raises an important implication for vaccine development against flagellated pathogens and may indicate one mechanism by which bacteria facilitate host's antibody responses to evade from host's innate bactericidal activities (Fig. 5).

### MATERIALS AND METHODS

### Construction of mammalian expression plasmids for vaccination

The *fliC* gene of PAO1 and *flaA* gene of PAK were PCR-amplified and ligated to pFLAG CMV5b (Sigma, St. Louis, MO) or pGACAG (17). In some cases, site-directed mutagenesis was performed to replace a target amino acid with alanine (L88A, R90A, Q97A, V404A, and F425A). The sequences of inserted DNA fragments were confirmed with Genetic Analyzer 310 (PE Applied Biosystems, Foster City, CA).

### Purification of recombinant proteins

Recombinant proteins were expressed in *E. coli* DH5α and purified as described (17). Contaminating endotoxin was removed using Detoxi-Gel (PIERCE, Rockford, IL). Finally, the amount of contaminated endotoxin was <0.003 ng/mg protein.

### Immunization schedule

Eight-week old female BALB/c mice were housed in animal facility under specific pathogen-free conditions. Mice were immunized with 50 µg of pGACAG-FliC WT, pGACAG-FliC R90A or pGACAG-FlaA by intramuscular electroporation using CUY21EDIT (NepaGene, Tokyo, Japan) (18). A booster immunization was given 3 weeks after the first immunization. Animal experiments were approved by the Institutional Animal Care and Welfare Committee.

### Immunoblotting analysis

IgGs from mouse sera were purified using Protein G sepharose columns (HiTrap Protein G HF, GE Healthcare Bio-Science AB). Specificity of IgG was analyzed with flagellar immunoblots prepared from PAO1 and PAK as described (19).

### ELISA

Serum antibody titer was measured by ELISA as described previously (20). TNF-α levels in the broncho-alveolar fluid were measured by ELISA according to the manufacturer's protocol (eBioscience, San Diego, CA).

### Luciferase assay

Luciferase assay was performed as described (21). Briefly, HEK293 cells (4 x 10⁴) were transiently transfected with 40 ng of either mouse or human TLR5 plus 5 ng of pNF-κ B-Luc (Stratagene, La Jolla, CA) and pTK-RL (Promega, Madison, WI). Forty-two hours after transfection, cells were stimulated with different concentrations of each recombinant flagellin for 6 hours in the presence or absence of purified IgG (10 µg/ml) from control or vaccinated mice. Luciferase assay was performed using Dual-Luciferase Reporter Assay System (Promega). Firefly luciferase activity was normalized to *Renilla* luciferase activity and depicted as relative luciferase activity.

### Protection test

Mice were anesthetized and challenged intranasally with 2 LD₅₀ doses of PAO1 or PAK. In some cases, rFliC (1 µg) was pre-incubated with 20 µg of anti-FliC WT or R90A IgG purified from immunized mice sera at 37°C for 30 minutes, and mice were inoculated with this mixture 4 hours before challenge. The mortality of the challenged mice was monitored for the subsequent 10 days.

### Quantification of P. aeruginosa and MPO activity in the lung

Two weeks after final immunization, mice were anesthetized and inoculated intranasally with 5 x 10⁵ CFU of PAO1 or PAK in 30 µl of PBS. Twenty four hours post infection, viable cell count or MPO activity in the lung was determined as described previously (22).
1. Shapiro, L. 1995. The bacterial flagellum: from genetic network to complex architecture. Cell 80:525-527.
2. Pier, GB., G Meluleni, and J.B. Goldberg. 1995. Clearance of Pseudomonas aeruginosa from the murine gastrointestinal tract is effectively mediated by O-antigen-specific circulating antibodies. Infect. Immun. 63:2818-2825.
3. Akerley, B.J., P.A. Cotter, and J.F. Miller. 1995. Ectopic expression of the flagellar regulon alters development of the Bordetella-host interaction. Cell 80:611-620.
4. Uematsu, S., M.H. Jang, N. Chevrier, Z. Guo, Y Kumagai, M. Yamamoto, H. Kato, N. Sougawa, H. Matsui, H. Kuwata, H. Hemmi, C. Coban, T. Kawai, K.J. Ishii, O. Takeuchi, M. Miyasaka, K. Takeda, and S. Akira. 2006. Detection of pathogenic intestinal bacteria by Toll-like receptor 5 on intestinal CD 11c+ lamina propria cells. Nat. Immunol. 7:868-874.
5. Applequist, S.E., E. Rollman, M.D. Wareing, M. Liden, B. Rozell, J. Hinkula, and H.G Ljunggren. 2005. Activation of innate immunity, inflammation, and potentiation of DNA vaccination through mammalian expression of the TLR5 agonist flagellin. J. Immunol. 175:3882-3891.
6. Mizel, S.B., A.N. Honko, M.A. Moors, PS. Smith, and A.P. West. 2003. Induction of macrophage nitric oxide production by Gram-negative flagellin involves signaling via heteromeric Toll-like receptor 5/Toll-like receptor 4 complexes. J. Immunol. 170:6217-6223.
7. Yu, Y, H. Zeng, S. Lyons, A. Carlson, D. Merlin, A.S. Neish, and A.T. Gewirtz. 2003. TLR5-mediated activation of p38 MAPK regulates epithelial IL-8 expression via posttranscriptional mechanism. Am. J. Physiol. 285:G282-290.
8. Gewirtz, A.T., T.A. Navas, S. Lyons, P.J. Godowski, and J.L. Madara. 2001. Cutting edge: bacterial flagellin activates basolaterally expressed TLR5 to induce epithelial proinflammatory gene expression. J. Immunol. 167:1882-1885.
9. Liaudet, L., C. Szabo, O.V Evgenov, K.G Murthy, P. Pacher, L. Virag, J.G Mabley, A. Marton, F.G. Soriano, M.Y Kirov, L.J. Bjertnaes, and A.L. Salzman. 2003. Flagellin from gram-negative bacteria is a potent mediator of acute pulmonary inflammation in sepsis. Shock 19:131-137.
10. Means, T.K., F. Hayashi, K.D. Smith, A. Aderem, and A.D. Luster. 2003. The Toll-like receptor 5 stimulus bacterial flagellin induces maturation and chemokine production in human dendritic cells. J. Immunol. 170:5165-5175.
11. Rhee, S.H., A.C. Keates, M.P. Moyer, and C. Pothoulakis. 2004. MEK is a key modulator for TLR5-induced interleukin-8 and MIP3alpha gene expression in non-transformed human colonic epithelial cells. J. Biol. Chem. 279:25179-25188.
12. Lee, S.E., S.Y Kim, B.C. Jeong, Y.R. Kim, S.J. Bae, O.S. Ahn, J.J. Lee, H.C. Song, J.M. Kim, H.E. Choy, S.S. Chung, M.N. Kweon, and J.H. Rhee. 2006. A bacterial flagellin, Vibrio vulnificus FlaB, has a strong mucosal adjuvant activity to induce protective immunity. Infect. Immun. 74:694-702.
13. Lanyi, B. 1970. Serological properties of Pseudomonas aeruginosa. II. Type-specific thermolabile (flagellar) antigens. Acta Microbiol. Acad Sci. Hung. 17:35-48.
14. Smith, K.D., E. Andersen-Nissen, F. Hayashi, K. Strobe, M.A. Bergman, S.L. Barrett, B.T. Cookson, and A. Aderem. 2003. Toll-like receptor 5 recognizes a conserved site on flagellin required for protofilament formation and bacterial motility. Nat. Immunol. 4:1247-1253.
15. Verma, A., S.K. Arora, S.K. Kuravi, and R Ramphal. 2005. Roles of specific amino acids in the N terminus of Pseudomonas aeruginosa flagellin and of flagellin glycosylation in the innate immune response. Infect. Immure. 73:8237-8246.
16. Fields, B.A., F.A. Goldbaum, X. Ysern, R.J. Poljak, and R.A. Mariuzza. 1995. Molecular basis of antigen mimicry by an anti-idiotope. Nature 374:739-742.
17. Saha, S., F. Takeshita, S. Sasaki, T. Matsuda, T. Tanaka, M. Tozuka, K. Takase, T. Matsumoto, K. Okuda, N. Ishii, K. Yamaguchi, D.M. Klinman, K.Q. Xin, and K. Okuda. 2006. Multivalent DNA vaccine protects mice against pulmonary infection caused by Pseudomonas aeruginosa. Vaccine 24:6240-6249.
18. Takeshita, F., T. Tanaka, T. Matsuda, M. Tozuka, K. Kobiyama, S. Saha, K. Matsui, K.J. Ishii, C. Coban, S. Akira, N. Ishii, K. Suzuki, D.M. Klinman, K. Okuda, and S. Sasaki. 2006. Toll-like receptor adaptor molecules enhance DNA-raised adaptive immune responses against influenza and tumors through activation of innate immunity. J. Virol. 80:6218-6224.
19. Price, B.M., J. Barten Legutki, D.R. Galloway, B.U. von Specht, L.B. Gilleland, H.E. Gilleland, Jr., and J. Staczek 2002. Enhancement of the protective efficacy of an oprF DNA vaccine against Pseudomonas aeruginosa. FEMS Immunol. Med Microbiol 33:89-99.
20. Sasaki, S., R.R. Amara, A.E. Oran, J.M. Smith, and H.L. Robinson. 2001. Apoptosis-mediated enhancement of DNA-raised immune responses by mutant caspases. Nat. Biotechnol. 19:543-547*.*
21. Takeshita, F., C.A. Leifer, I. Gursel, K.J. Ishii, S. Takeshita, M. Gursel, and D.M. Klinman. 2001. Cutting edge: Role of Toll-like receptor 9 in CpG DNA-induced activation of human cells. J. Immunol. 167:3555-3558.
22. Schneider, T., and A.C. Issekutz. 1996. Quantitation of eosinophil and neutrophil infiltration into rat lung by specific assays for eosinophil peroxidase and myeloperoxidase. Application in a Brown Norway rat model of allergic pulmonary inflammation. J. Immunol. Methods 198:1-14*.*

### INDUSTRIAL APPLICABILITY

The present invention is applicable to the development of vaccines, in particular vaccines against flagellated pathogens. The vaccine of the present invention is effective against *P. aeruginasa* causing nosocomial infection.

Further, antibodies induced against the flagellin mutant of the present invention are applicable to treatment of infection with flagellated bacteria, in particular *P. aeruginosa.*

All the publications, patents and patent applications cited herein are incorporated herein by reference in their entirety.

### SEQUENCE LISTING FREE TEXT

<SEQ ID NO: 1>
   SEQ ID NO: 1 shows the nucleotide sequence of *P. aeruginosa* (PAOI)-derived wild-type flagellin FliC.
<SEQ ID NO: 2>
   SEQ ID NO: 2 shows the amino acid sequence of *P. aeruginosa* (PAO1)-derived wild-type flagellin FliC.
<SEQ ID NO: 3>
   SEQ ID NO: 3 shows the nucleotide sequence of mutant flagellin FliC R90A.
<SEQ ID NO: 4>
   SEQ ID NO: 4 shows the amino acid sequence of mutant flagellin FliC R90A.
<SEQ ID NO: 5>
   SEQ ID NO: 5 shows the nucleotide sequence of *P. aeruginosa* (PAK)-derived wild-type flagellin FlaA.
<SEQ ID NO: 6>
   SEQ ID NO: 6 shows the amino acid sequence of *P. aeruginosa* (PAK)-derived wild-type flagellin FlaA.

## Claims

1. A flagellin mutant into which a mutation has been introduced at least at one site in the Toll-like receptor 5 activation domain of a corresponding wild-type flagellin to thereby attenuate the ability to activate Toll-like receptor 5.

2. The flagellin mutant according to claim 1, wherein the wild-type flagellin is derived from a flagellated pathogen.

3. The flagellin mutant according to claim 2, wherein the flagellated pathogen is *Pseudomonas aeruginosa.*

4. The flagellin mutant according to any one of claims 1 to 3, wherein the wild-type flagellin is flagellin FlaA or FliC.

5. The flagellin mutant according to claim 4, wherein the wild-type flagellin is wild-type flagellin FliC having the amino acid sequence as shown in SEQ ID NO: 2 and the Toll-like receptor 5 activation domain includes a region spanning from position 71 to position 97 of the amino acid sequence as shown in SEQ ID NO: 2.

6. The flagellin mutant according to claim 5, which has the amino acid sequence as shown SEQ ID NO: 2 wherein arginine at position 90 is substituted with another amino acid.

7. The flagellin mutant according to claim 6, wherein another amino acid is alanine or glycine.

8. The flagellin mutant according to claim 1, which is a protein selected from the following (a), (b) and (c):
(a) a flagellin mutant protein having the amino acid sequence as shown in SEQ ID NO: 2 wherein arginine at position 90 is substituted with alanine;
(b) a flagellin mutant protein which has the amino acid sequence of the protein of (a) wherein one or more amino acids other than alanine at position 90 are deleted, substituted or added, and whose Toll-like receptor 5 activation ability is attenuated compared to the corresponding ability of wild-type flagellin FliC having the amino acid sequence as shown in SEQ ID NO: 2; and
(c) a flagellin mutant protein which is encoded by a DNA that hybridizes to a complementary DNA to a DNA encoding the protein of (a) under stringent conditions, and whose Toll-like receptor 5 activation ability is attenuated compared to the corresponding ability of wild-type flagellin FliC having the amino acid sequence as shown in SEQ ID NO: 2.

9. The flagellin mutant according to claim 8, wherein the DNA encoding the protein of (a) has the polynucleotide sequence as shown in SEQ ID NO: 3.

10. A DNA encoding the flagellin mutant according to any one of claims 1 to 9.

11. A vector comprising the DNA according to claim 10.

12. A transformant comprising the vector according to claim 11.

13. A method of preparing a flagellin mutant, comprising culturing the transformant according to claim 12.

14. A vaccine comprising the flagellin mutant according to any one of claims 1 to 9, the DNA according to claim 10 or the vector according to claim 11.

15. The vaccine according to claim 14, which is against a flagellated pathogen.

16. The vaccine according to claim 15, wherein the flagellated pathogen is *Pseudomonas aeruginosa.*

17. The vaccine according to any one of claims 14 to 16, which is used for protection against cystic fibrosis and/or protection of medically compromised patients.

18. An antibody induced against the flagellin mutant according to any one of claims 1 to 9.

19. A pharmaceutical composition comprising the antibody according to claim 18.
